(19) Europäisches Patentamt
European Patent Office
Office européen des brevets

(11) Publication number: 0 214 556 B1

(12) EUROPEAN PATENT SPECIFICATION

(45) Date of publication of patent specification: 22.11.90

(21) Application number: 86111890.9

(22) Date of filing: 28.08.86

(51) Int. Cl.[5]: C 07 D 491/147, C 07 D 495/14, C 07 D 471/14, C 07 D 455/06, C 07 D 513/14, A 61 K 31/435

# (54) Substituted hexahydro-arylquinolizine derivatives, processes for their preparation and pharmaceutical composition containing them.

(30) Priority: 03.09.85 US 771927

(43) Date of publication of application: 18.03.87 Bulletin 87/12

(45) Publication of the grant of the patent: 22.11.90 Bulletin 90/47

(84) Designated Contracting States: CH DE FR GB IT LI NL

(56) References cited:
EP-A-0 154 142
AU-A-3 822 085
GB-A-1 435 573
GB-A-2 106 909

IUPAC Nomenclature of Organic Chemistry (Pergamon Press) (1979)

(73) Proprietor: MERCK & CO. INC.
126, East Lincoln Avenue P.O. Box 2000
Rahway New Jersey 07065-0900 (US)

(72) Inventor: Huff, Joel R.
738 Bergey Mill Road
Lederach, PA. 19450 (US)
Inventor: Baldwin, John J.
621 Gypsy Hill Circle
Gwynedd Vally, PA 19437 (US)
Inventor: Vacca, Joseph P.
766 Eisenhauer Drive
Telford, PA 18969 (US)
Inventor: Guare, James P., Jr.
Kumry Road
Quakertown, PA. 18951 (US)
Inventor: Sakurai, Yojiro MSD(Japan)Co.Ltd.
Kowa Building 16 Annex 9-20 Akasaka 1-chome
Minato-ku Tokyo 107 (JP)

(74) Representative: Blum, Rudolf Emil Ernst et al
c/o E. Blum & Co Patentanwälte Vorderberg 11
CH-8044 Zürich (CH)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 214 556 B1

## Description

Background of the Invention

This invention is concerned with novel substituted hexahydro-arylquinolizines and salts thereof. Said new compounds and the pharmaceutically acceptable salts thereof. Said new compounds and the pharmaceutically acceptable salts thereof are selective α-adrenoceptor antagonists are of value in conditions where selective antagonism of the $α_2$-adrenoceptor is desirable for example as antidepressant, antihypertensive, ocular antihypertensive, antidiabetic, antiobesity agents, platelet aggregation inhibitors or modifiers of gastrointestinal motility. It also relates to processes for preparing the novel compounds and pharmaceutical compositions comprising the novel compounds which compositions are used for antagonizing $α_2$-adrenoceptors.

The concept that the complex clinical state of depression is linked to a functional deficiency of monoamines in the central nervous system is now widely accepted. Numerous biochemical and clinical observations support the proposal that many forms of depressive illness are associated with reductions in adrenergic activity at functionally important sites in the brain. Thus, classical antidepressive drugs, such as amitriptyline and imipramine, are believed to act by blocking the neuronal reuptake of norepinephrine and/or serotonin, thereby enhancing the availability of the monoamines as neurotransmitters.

In addition to $α_1$-adrenergic receptors which mediate postsynaptic responses to the neurotransmitter, norepinephrine, other adrenergic receptors are present at or near sympathetic terminals. These latter receptors, $α_2$-adrenergic receptors, form part of a negative feedback system which modulates noradrenergic neurotransmission by controlling the impulse-induced release of norepinephrine from presynaptic terminals. Activation of $α_2$-adrenergic receptors results in a decrease in the amount of norepinephrine normally released from the nerve terminals by nerve impulses while antagonism of $α_2$-adrenergic receptors increases norepinephrine release. Therefore, molecules that block $α_2$-adrenergic receptors afford an alternate approach to enhancement of noradrenergic function and the treatment of depression associated with an absolute or relative deficiency of adrenergic function.

$α_2$-Adrenergic receptor antagonism is also associated with antidiabetic, antihypertensive, ocular antihypertensive, antiobesity, platelet aggregation inhibition activity, and modification of gastrointestinal motility.

Description of the Prior Art

In the British patent 1,435,573 of John Wyeth and Brother, Ltd. there are described hydrogenated arylquinolizines, in which the condensed aryl group has to be the heteroaromatic indole structure. Said compounds are substituted with an acylated amino group which is bonded to the hydrogenated arylquinolizine structure, for example in the position 2 thereof. The compounds disclosed in said British patent have a hypotensive activity.

In the British patent 2,106,909 of John Wyeth and Brother, Ltd. there are described hexahydroarylquinolizine which are $α_2$-adrenoceptor antagonists. To the carbon atom in the position 2 of said hexahydroarylquinolizines there is bonded the nitrogen atom of a substituted amino group.

In the Australian patent abstract AU—A—38 220/85 of Merck & Co. there are described hydrogenated arylquinolizines, in which the aryl is an aromatic group or a heteroaromatic group. To the carbon atom in the position 2 of said hydrogenated arylquinolizines there has to be bonded the nitrogen atom of an acylated amino group, which acylated amino group has the folowing structure:

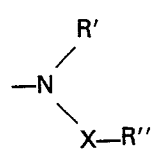

wherein

X is 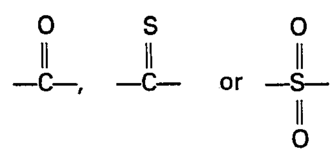

R′ is hydrogen, acyl or optionally substituted $C_{1-6}$alkyl and

R″ is an aliphatic, cycloaliphatic, aromatic or heteroaromatic radical, a hydroxy or alkoxy group or can also have some other meanings.

It said hydrogenated arylquinolizines, accordingly, the nitrogen atom of the acylated amino group has to be directly bonded to the carbon atom of the hydrogenated quinolizine nucleus. Said compounds are selective $α_2$-adrenoceptor antagonists.

It was the aim of the present invention to provide new hexahydro-arylquinolizine-derivatives, which differ as to the substituent in the position 2 of the quinolizine nucleus from the corresponding prior art compound and which are selective $α_2$-adrenoceptor antagonists.

Description of the Invention

One object of the present invention are compounds of structural formula I

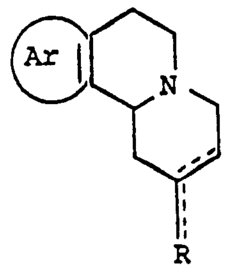

I

or salts of the compounds of formula I wherein Ar represents an aromatic or heteroaromatic group selected from

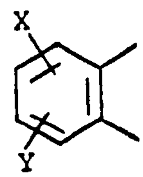

benzo-

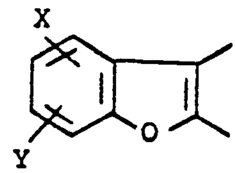

benzofuro-

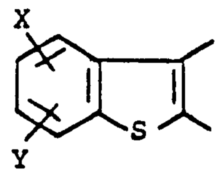

benzothieno-

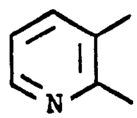

pyridino-

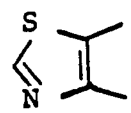

thiazolo-

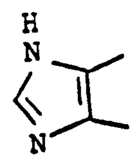

imidazo-

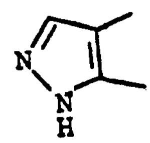

pyrazolo-

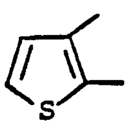

thieno-

and

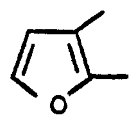

furo-

wherein

X and Y are independently:

1) hydrogen,
2) halo,
3) hydroxy,
4) $C_{1-3}$alkoxy, or
5) $C_{1-6}$alkyl;

R is

1) $COOR^1$ wherein

$R^1$ is hydrogen or $C_{1-5}$alkyl, either straight or branched chain;

2)

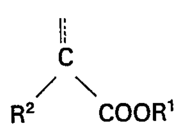

wherein

$R^1$ is as defined in (1) and

$R^2$ is a) hydrogen,

b) $C_{1-5}$alkyl, or

c) allyl

3)

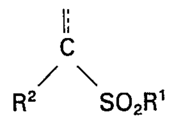

wherein

$R^1$ and $R^2$ are as defined in (2)

4)

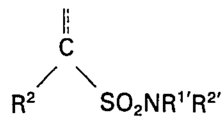

wherein

$R^{1'}$ and $R^{2'}$ have the same meaning as $R^1$ and $R^2$ in (2) or $R^{1'}$and $R^{2'}$ form together with the nitrogen atom to which they are bonded a 5- or 6-membered heterocycle which is selected from the group comprising pyrrolidino, morpholino, piperidino, and piperazino:

5)

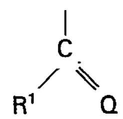

wherein

Q is oxygen or a group $=N—OR^1$ wherein $R^1$ has the same meaning as in (1)

6)

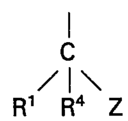

wherein

Z is a) $—OR^3$ or $—SR^3$

wherein

$R^3$ is

i) H,

ii) $C_{1-5}$alkyl, unsubstituted or substituted with

1) —OH,

2) $—COOR^1$,

3) $—SO_2R^2$,

4) $—N(R^2)SO_2R^2$,

wherein

$R^1$ and $R^2$ are as defined in (2) or

b) $—NR^2R^3$

wherein

$R^2$ is as defined in (2) and

$R^3$ is as defined in (a) above

$R^4$ is a) hydrogen

b) $C_{1-5}$alkyl, or

$C_{1-5}$alkylidene or allyl and

$R^1$ is as defined in (2) and the broken lines represent possible double bonds.

The compounds of formula I are intended to be used as active ingredient of pharmaceutical compositions and therefore salts of the compounds of formula I are preferably pharmaceutically acceptable salts.

The pharmaceutically acceptable salts include the pharmaceutically acceptable acid addition salts. Acids useful for preparing these acid addition salts include, inter alia, inorganic acids, such as the hydrohalic acids (e.g., hydrochloric and hydrobromic acid), sulfuric acid, nitric acid, and phosphoric acid,

and organic acids such as maleic, fumaric, tartaric, citric, acetic, benzoic, 2-acetoxybenzoic, salicylic, succinic, theophylline, 8-chlorotheophylline, p-aminobenzoic, p-acetamidobenzoic, methanesulfonic, or ethanedisulfonic acid.

The novel compounds of formula I wherein R is attached by a single bond are depicted herein as having the configuration in which the hydrogen at C-12b and R at C-2 are *trans*

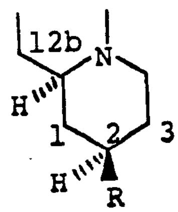

and it is the more preferred diastereomer for $\alpha_2$-adrenoceptor blockage activity. However, the isomers having the configuration in which the hydrogen at C-12b and R at C-2 are *cis* are also active $\alpha_2$-adrenoceptor blockers and are considered to be within the scope of formula I. Each of the 2RS,12bSR and 2RS,12bRS-configurational isomers are racemates capable of being resolved into dextrorotatory and levorotatory enantiomers. Formula I includes these pure enantiomers as well as all mixtures thereof, especially the racemates. However, the (12bS)-enantiomer is preferred.

It is also preferred that Ar be X,Y-benzo-, X,Y-benzo[b]furo; or X,Y-benzo[b]thieno-. It is even more preferred that Ar be benzo[b]furo.

Furthermore, in the compounds of the general formula I and in the preferred compounds mentioned above the radical R is preferably one of the following groups

$$—CH_2—CO_2R^1,\quad CH_3\overset{|}{C}HCO_2R^1,\quad R^1—\overset{|}{C}{=}NOR^1,\quad R^1(R^4)\overset{|}{C}—OH,$$

$$R^1—\overset{|}{C}H—O(CH_2)_2OH,\ \text{or}\ \ R^1(R^4)\overset{|}{C}—O—(CH_2)_2OH$$

wherein

$R^1$ and $R^4$ are independently hydrogen atoms or alkyl groups having 1—5 carbon atoms, and in the above stated groups of formulae

$$—CH_2—CO_2R^1,\ CH_3\overset{|}{C}HCO_2R^1,\ R^1—\overset{|}{C}{=}NOR^1,$$

the radical $R^1$ is preferably an alkyl group having 1—5 carbon atoms.

Preferred meanings of the radical $R^1$ and $R^4$ in the above stated formulae are methyl groups.

A further object of the present invention are the processes for the preparation of the claimed compounds of formula I and the salts thereof.

Compounds of formula If

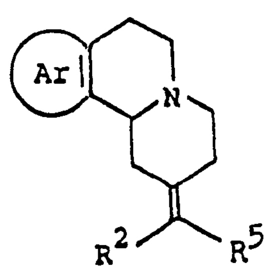

If

wherein

$R^5$ is $—COOR^1$

$—SO_2R^1$ or

$—SO_2NR^{1'}R_2'$

wherein

Ar, $R^1$, $R^2$, $R^{1'}$ and $R^{2'}$ have the same meaning as in formula I can be prepared by reacting a 2-oxoquinolizine of formula II

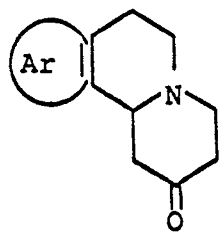

II

with a Wittig reagent, preferably of formula

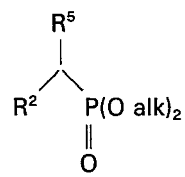

in an inert aprotic solvent in the presence of a strong base to yield the compounds of formula If which are isolated as free compounds or as salts thereof. Examples of inert aprotic solvents in which the reaction with the Wittig reagent is performed are ethers, for example tetrahydrofurane, diethyl ether, glyme or tetrahydropyran and examples for strong bases which are used in said reaction are KH or n-butyl lithium. Said reaction is preferably performed in an inert atmosphere at about −70°C to 0°C for about 0.5 to about 3 hours followed by spontaneous warming to room temperature over about 16 hours.

Inventive compounds of formula Ig

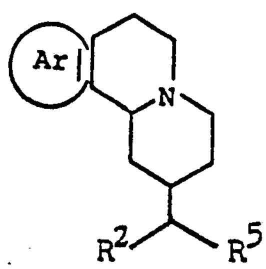

Ig

wherein

$R^2$ and $R^5$ are as defined in the compounds of formula If can be prepared by reducing the compounds of formula If. Said reduction reaction is readily performed by hydrogenation in the presence of a noble metal catalyst, like palladium on carbon, platinum oxide or other noble metal catalysts. The hydrogenation is preferably performed in a lower alkanol at a pressure of hydrogen of about 2,81 atm. (40 psi) until the requisite amount of hydrogen is absorbed; usually about 0.5 to about 3 hours.

The compounds having the following formula Ia

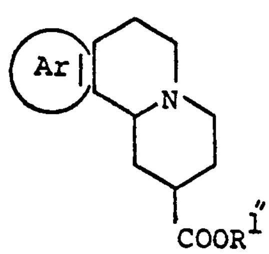

Ia

or salts thereof wherein

Ar is as defined in formula I and

$R^{1''}$ is $C_{1-5}$alkyl

can be prepared by reacting a compound of formula II

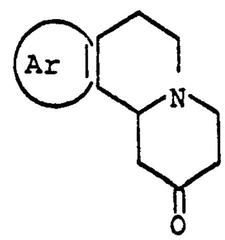

II

with 2-trimethylsilyl-1,1-dithiane of formula III

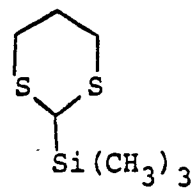

III

in the presence of an etheral solvent and a strong base yielding a 1,3-dithian-2-ylidene derivative of formula IV

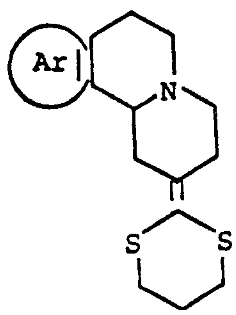

IV

Thereafter said intermediate product of formula IV is reacted with a $C_{1-5}$ alkanol of formula $R^{1''}$———OH, which alkanol is saturated with hydrogen chloride yielding the desired final products of formula Ia, which are isolated in the free form or as salts thereof.

Examples for etheral solvents in which the reaction with the 2-trimethylsylyl-1,3-dithiane of formula III is conducted are for instance tetrahydrofurane, tetrahydropyran, or diethyl ether. The reaction is conducted in the presence of a strong base such as potassium or sodium hydride, or a metal organic compound such as n-butyl lithium, preferably at about −40°C to −10°C followed by spontaneous warming to room temperature. After the isolation of the 1,3-dithian-2-ylidene derivative of formula IV said compound is treated with the alkanol having 1—5 carbon atoms, for example with ethanol, which is saturated with hydrogen chloride. Said reaction is preferably conducted at about 25° to 75°C for about 1 to 5 hours.

The compounds of formula Ib

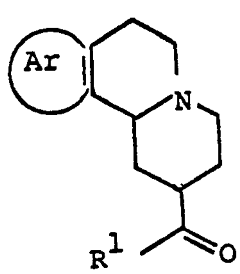

Ib

or salts thereof wherein

Ar and $R^1$ have the same meaning as in formula I can be prepared by reacting a compound of formula II

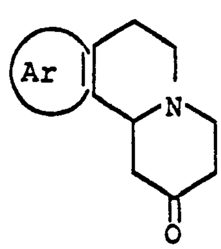

II

either with a phosphorus compound of formula

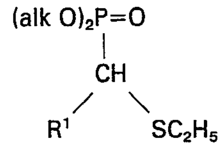

wherein

$R^1$ is hydrogen or $C_{1-5}$alkyl, in order to produce corresponding compounds of formula Ib wherein $R^1$ is hydrogen or an alkyl group having 1—5 carbon atoms or those compounds of formula Ib wherein $R^1$ is hydrogen can be alternatively prepared by reacting the compounds of formula II with a phosphorus compound of formula

$$Ph_3P^+—CH_2—O—CH_3Cl^-.$$

The intermediate products which are produced through the reaction with the above stated phosphorus compounds are then hydrolized to yield the compounds of formula Ib.

The reaction of the 2-oxoquinolizine of formula II with (1-ethylthio)alkyldialkylphosphonate having the above stated formula is performed under Wittig conditions, i.e. preferably in the presence of a strong base, such as KH or n-butyl lithium in an inert aprotic solvent, preferably an ether, for example tetrahydrofurane, diethyl ether, glyme or tetrahydrofuran.

The reaction of the 2-oxoquinolizine of formula II with the triphenylphosphoro compound of the above stated formula is as well conducted under the conditions of a Wittig reaction in an aprotic solvent, preferably an ethereal solvent, like tetrahydrofurane, diethyl ether, glyme or tetrahydropyran or in an aromatic solvent, like benzene or toluene. Said reaction is preferably performed at a temperature of about −10°C to +5°C over about 0.5 to 3 hours.

The intermediate products which are produced through the reaction with the phosphorus compounds are then hydrolized to yield the compounds of formula Ib. Said hydrolization is performed with water and saturated aqueous ammonium chloride solution, provided that the used phosphoro compound was the (1-ethylthio)alkyldialkylphosphonate of the above stated formula.

The acid hydrolysis of the intermediate product which is produced through the reaction with the triphenylphosphoro compound of the above stated formula is an enol ether having the following formula

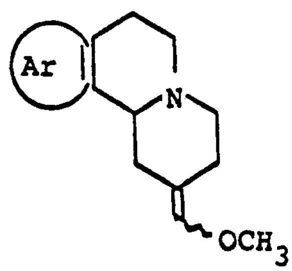

The hydrolysis, using an acid, of said enol ether is accomplished by treatment with dilute mineral acid such as hydrochloric acid at about room temperature, although the temperature is not critical. In the resulting compound of formula Ib the radical $R^1$ is hydrogen, i.e. the corresponding compound is a formyl compound.

The oximes of formula Id

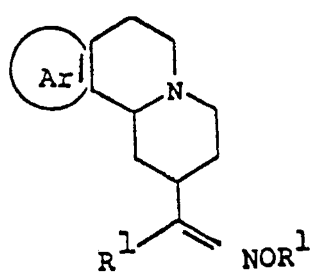

Id

wherein $R^1$ is hydrogen or alkyl having 1—5 carbon atoms can be prepared by reacting the keto compounds, respectively formyl compounds of formula Ib with a hydroxylamine or alkoxyamine of formula

$$H_2NOR^1$$

in the presence of an organic base, such as pyridine, optionally diluted with a lower-alkanol such as ethanol.

The compounds of formula Ie

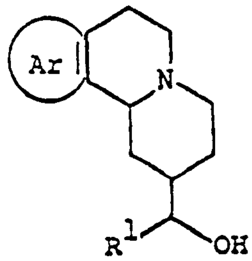

Ie

wherein $R^1$ is hydrogen or alkyl having 1—5 carbon atoms can be prepared by reducing the keto compounds or formyl compounds of formula Ib. Said reduction is preferably performed with a complex metal hydride, for example lithium aluminum hydride, preferably in an ethereal solvent at about −10°C to about +30°C.

The compounds of formula Ic

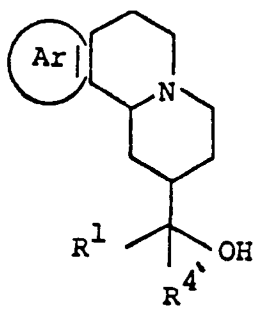

Ic

wherein

$R^1$ is hydrogen or alkyl having 1—5 carbon atoms and

$R^{4'}$ is $C_{1-5}$alkyl, $C_{1-5}$alkylidene or allyl can be prepared by reacting the keto compounds or formyl compounds of formula Ib with a Grignard reagent of formula

$$R^{4'}\ MgHal$$

in which

$R^{4'}$, is as defined in formula Ic and

Hal is haline.

Said reaction with the Grignard reagent is performed under conditions usual for the performance of a Grignard reaction, preferably in an ethereal solvent at a temperature of about −10°C to +10°C for about 1—4 hours.

The compounds of formula

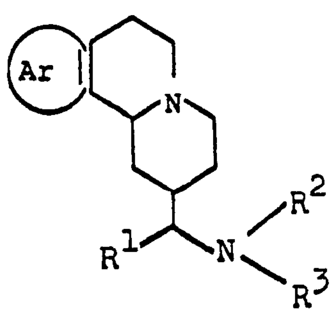

wherein

$R^1$ is hydrogen or alkyl having 1—5 carbon atoms

$R^2$ is hydrogen, alkyl having 1—5 carbon atoms or alkenyl with up to 5 carbon atoms and

$R^3$ is hydrogen, alkyl having 1—5 carbon atoms which is unsubstituted or substituted with the substituents defined for in connection with formula I can be prepared by submitting the keto compounds or

formyl compounds of formula I to a reductive amination with an amine of formula

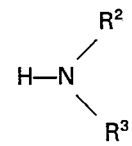

According to said process of the reductive amination accordingly the corresponding amino compound wherein

$R^2$ is hydrogen and

$R^3$ is a substituted alkyl group of formula

$$-CH_2-CH_2-OH$$

can be prepared by treating the keto compound or formyl compound of formula Ib with ethanolamine and sodium cyanoborohydride ($NaCNBH_3$) in methanolic hydrogen chloride at about 15 to 30°C over a period of about 10 to 30 hours.

The reaction steps performed in order to produce a corresponding amino compound wherein $R^1$ and $R^3$ are hydrogen starting with the compounds of formula II are illustrated with the following reaction scheme:

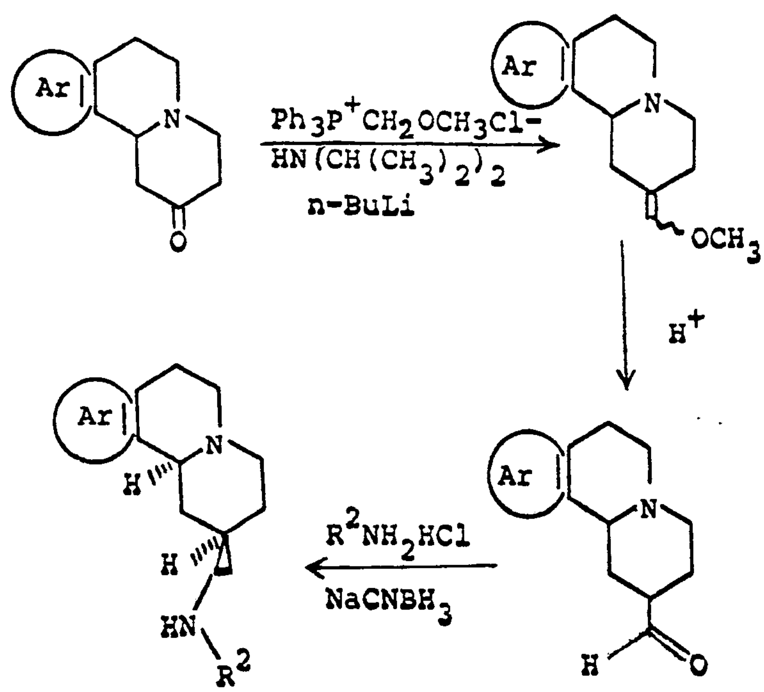


According to said reaction scheme the first step, i.e. the Wittig reaction, is preferably conducted under the preferred reaction conditions outlined before. The acidic hydrolysis of the enol ether is preferably performed with a dilute mineral acid, for instance hydrochloric acid at a temperature which is not critical, for instance room temperature. The reductive amination of the resulting formyl compound is then preferably performed maintaining the reaction conditions which were explained in connection with the reductive amination performed with ethanol amine.

The compounds of formula Ih

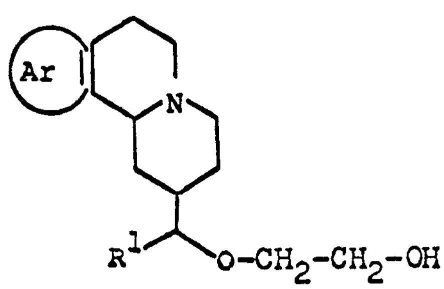

Ih

can be prepared by treating compounds of formula Ie

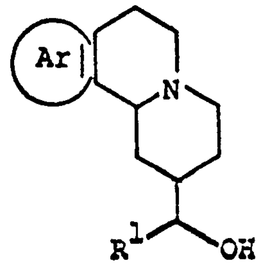

Ie

with sodium hydride or potassium hydride or phenyl lithium followed by ethyl bromoacetate yielding the ethoxycarbonylmethyl ether which on reduction with sodium borohydride provides the compounds of formula Ih. The condensation with ethyl bromoacetate is preferably conducted at about −10°C to +10°C, preferably 0°C in an inert protic solvent such as a benzene/DMF mixture over a period of about 1 to 5 hours. The borohydride reduction is preferably conducted in refluxing t-butanol for about 0.5 to 2 hours.

The compounds of the following formula

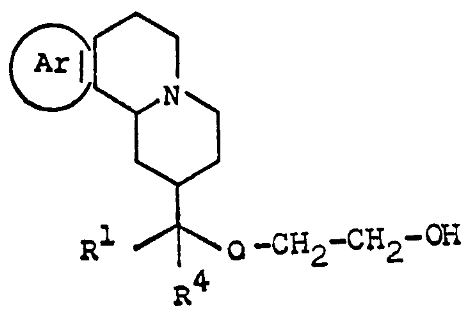

are most readily prepared by preparing the ethylene ketal of the 2-alkanoyl-quinolizine by treating the alkanoyl compound with ethylene glycol, followed by treatment of the ketal with a Grignard reagent of formula $R^4MgBr$. The ethylene ketal is formed by treating a mixture of the ethylene glycol and the 2-alkanoyl-quinolizine with boron trifluoride etherate in a chlorinated alkane such as methylene chloride, chloroform or the like at about 15 to 35°C for about 10 to 24 hours in the absence of light. The Grignard reaction is conducted in an aromatic hydrocarbon such as benzene, toluene or the like at about 70—90°C, conveniently in refluxing benzene.

If the hemithioketal prepared with 2-mercaptoethanol as described above for preparation of the ethylene ketal is added to a cold (0°C) solution of aluminum chloride and lithium aluminum hydride in ether followed by refluxing for 2 hours followed by acidification, there are produced the compounds wherein R is

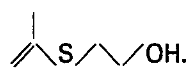

A further object of the present invention are pharmaceutical compositions for the treatment of depression, hypertension, ocular hypertension, diabetes, obesity or platelet aggregation or for modifying gastrointestinal motility, which pharmaceutical compositions are characterized in that they comprise as pharmaceutically effective ingredient a compound of formula I or a pharmaceutically acceptable salt thereof.

Said pharmaceutical compositions preferably contain as active ingredient the preferred compounds of formula I defined before or a pharmaceutically acceptable salt or said compounds and optionally furthermore a pharmaceutical carrier material.

Said pharmaceutical compositions can be used for treating depression, ocular hypertension, hypertension, diabetes, obesity or platelet aggregation, or modifying gastrointestinal motility.

The pharmaceutical compositions are preferably administered in such doses that the inventive compound or the pharmaceutically acceptable salt thereof is administered in an amount ranging from about 0.01 to about 20 mg per kg of body weight per day, preferably from about 0.1 to about 10 mg per kg of body weight per day in a single dose or in 2 to 4 divided doses.

The pharmaceutical compositions for the treatment of depression can contain the inventive compounds of formula I as sole, pharmaceutically active ingredients or in combination with other antidepressants such as amitriptyline, imipramine or other norepinephrine or serotonin reuptake inhibitor or a monoamine oxidase inhibitor.

The pharmaceutical compositions can be formulated so that they can be administered orally, intraperitoneally, subcutaneously, intramuscularly, transdermally or intravenously. They are preferably

administered orally, for example in the form of tablets, troches, capsules, elixirs, suspensions, syrups, wafers, chewing gum, or the like prepared by art recognized procedures. The amount of active compound in such therapeutically useful compositions or preparations is such that a suitable dosage will be obtained.

## Example 1

(2RS,12bSR)-Methyl (1,3,4,6,7,12b-hexahydro-2H-benzo[b]furo[2,3-a]quinolizin-2-yl) acetate Hydrochloride.

*Step A:* Preparation of 3-Cyanomethylbenzo[b]furan

To a suspension of 2.64 gms (0.11 mole) of oil free sodium hydride in 200 mL of tetrahydrofuran (THF) was added dropwise a solution of 19.47 gms (0.11 mole) of diethylcyanomethylphosphonate in 75 mL of THF. After the $H_2$ evolution had ceased, a solution of 13.4 g (0.1 mole) of 3-(2H)-benzo[b]-furanone in 100 mL of THF was added. The solution was heated at 70°C for 1.5 hrs, cooled, and poured into 500 mL of 5% HCl, and washed with ether. The ether phase was washed with brine, dried ($MgSO_4$), filtered and concentrated to give 15.4 g of a dark oil. The product was distilled at 96—100°C at a pressure of 0.075 mm Hg (9.9992 Pa) to give 10.85 g of a yellow oil which crystallized upon standing.

*Step B:* Preparation of 2-(3-benzo[b]furanyl)ethylamine

A solution of 3.97 g (0.025 mole) of 3-cyanomethylbenzo[b]furan in 200 mL of diethyl ether was slowly added to a refluxing suspension of 3.84 g (0.1 mole) of lithium aluminum hydride in 400 mL of ether. The reaction was heated 3 hrs., cooled and water was slowly added. The suspension was filtered through a pad of filter aid and the filtrate was evaporated to give 3.2 g of oily product. The hydrochloride salt has m.p. 183—185°C.

*Step C:* Preparation of 3-(2-Formamidoethyl)benzo[b]furan

A solution of 2.35 g (0.015 mole) of 2-(3-benzo[b]furanyl)ethylamine and 5 mL of ethyl formate was heated at 60°C for 3 hours, poured into 2*N* HCl and washed with methylene chloride which in turn was washed with 5% sodium hydroxide (w/v), dried ($MgSO_4$), filtered and concentrated to give 2.70 g of product.

*Step D:* Preparation of 3,4-dihydrobenzo[b]furo[2,3-c]pyridine

2.28 Grams (0.012 mole) of 3-(2-formamidoethyl)benzo[b]furan was added to 28 g of polyphosphoric acid which was preheated to 100°C. After 1—1.5 hours, the reaction mixture was poured onto ice and the residues were washed with water. The polyphosphoric acid was dissolved in water, filtered through a pad of celite and made basic with concentrated ammonia. A precipitate was collected and dried to give 1.45 g of product, m.p. 170—171°C.

*Step E:* Preparation of (12bRS)-1,3,4,6,7,12b-Hexahydrobenzo[b]furo[2,3-a]quinolizin-2-one

To a solution of 12 g (0.070 mol) of 3,4-dihydrobenzo[b]furo[2,3-c]pyridine dissolved in 500 mL of acetonitrile at 60°C was added 20 g (0.140 mol) of 2-trimethylsiloxy-1,3-butadiene followed by 13.6 g (0.10 mol) of anhydrous zinc chloride. The mixture was heated at 60°C for 1.5 hour, cooled to 25°C, and 30 mL of 5% HCl was added and stirred 10 minutes. 40% Sodium hydroxide was added until the reaction was basic; 200 mL of water was added; and the acetonitrile layer was separated. The aqueous layer was filtered through celite and washed with ether. The combined organic layers were dried ($Na_2SO_4$), filtered, and concentrated to a brown residue which was chromatographed (silica, ethyl acetate/hexane (1:1)) to give 8.2 g of product, m.p. 108—9°C.

Resolution of (12bSR)-1,3,4,6,7,12b-hexahydrobenzo[b]furo[2,3-a]quinolizin-2-one

A solution of (−)-di-p-toluoyl-L-tartaric acid monohydrate (25.9 g) in 100 mL of ethyl acetate was mixed with a solution of (12bSR)-1,3,4,6,7,12b-hexahydrobenzo[b]furo[2,3-a]quinolizin-2-one (15.5 g) in 700 mL of ethyl acetate and allowed to stand 12—78 hours. The mixture was filtered to yield 21 g of the di-p-toluoyl-L-tartrate salt of the amine. The free base was liberated by partitioning between aqueous $Na_2CO_3$ and ethyl acetate ($[\alpha]_D$ = ca. −79°; C=0.001.; $CHCl_3$). The diastereomeric salt of this material was again prepared following the above procedure. The collected di-p-toluoyl-L-tartrate salt was partitioned between ethyl acetate and aqueous $Na_2CO_3$, dried ($Na_2SO_4$), filtered, treated with charcoal, filtered and evaporated to yield 5.4 g (35%) of (12bS)-1,3,4,6,7,12b-hexahydrobenzo[b]furo[2,3-a]quinolizin-2-one; $[\alpha]_D$ = −84°; (C=0.001, $CHCl_3$).

The (12bR)-1,3,4,6,7,12b-hexahydrobenzo[b]furo[2,3-a]quinolizin-2-one was obtained by substituting (+)-di-p-toluoyl-D-tartaric acid monohydrate for (−)-di-p-toluoyl-L-tartaric acid in the above procedure to provide product with $[\alpha]_D$ = +84° (C=0.001, $CHCl_3$).

*Step F:* Preparation of E and Z Methyl (1,3,4,6,7,12b-hexahydro-2H-benzo[b]furo[2,3-a]quinolizin-2-ylidene) acetate Hydrochloride.

Methyl-(dimethyl phosphonoacetate) (728 mg; 4 mmol) in 2 mL of dry THF was added to a suspension of KH (668 mg of a 24% oil dispersion; 4 mmol) in 2 mL of dry THF at 0°C under an atmosphere of nitrogen. After 30 minutes at 0°C, 1,3,4,6,7,12b-hexahydro-2H-benzo[b]furo[2,3-a]quinolizin-2-one (241 mg; 1 mmol)

was added, and the reaction mixture was stirred at room temperature for 18 hours. The mixture was partitioned between $CH_2Cl_2$ and water. The organic layer was separated and evaporated to an oil which was chromatographed over silica gel, eluting with 15% ethyl acetate/hexane. The E olefin (110 mg) and Z olefin (100 mg) were obtained as oils which were converted to HCl salts melting at 218—219°C and 220—221°C respectively.

*Step G:* Preparation of (2RS,12bSR)-Methyl (1,3,4,6,7,12b-hexahydro-2H-benzo[b]furo[2,3-a]quinolizin-2-yl) acetate Hydrochloride.

A 1:1 mixture of the E and Z olefins from Step F (700 mg) and 10% Pd on carbon (75 mg) in 100 mL of ethanol were hydrogenated at 40 psi (2.8 × $10^5$ Pa) for 1.5 hours. After separating the catalyst by filtration, the filtrate was evaporated to dryness. The resulting oil was chromatographed over silica gel, eluting with 40% ethyl acetate/hexane. The product was collected as an oil (370 mg) which crystallized upon standing. The free base was converted to the HCl salt, m.p. 194—195°C.

Starting with optically active 1,3,4,6,7,12b-hexahydro-2H-benzo[b]furo[2,3-a]quinolizin-2-one and employing the procedures in Steps F and G, the enantiomerically pure products are obtained.

Example 2

(2RS,12bSR,2′RS) and (2RS,12bSR,2′SR)-Ethyl 2′-(1,3,4,6,7,12b-hexahydro-2H-benzo[b]furo[2,3-a]quinolizin-2-yl) propionate Hydrochloride

*Step A:* Preparation of E and Z Ethyl 2′-(1,3,4,6,7,12b-hexahydro-2H-benzo[b]furo[2,3-a]quinolizin-2-ylidene) propionate.

Employing the procedure described in Step F of Example 1, and substituting triethyl 2-phosphonopropionate for trimethyl phosphonoacetate, the product was obtained as a mixture of E and Z geometric isomers which were separated by chromatography over silica gel, eluting with 20% ethyl acetate/hexane.

*Step B:* Preparation of (2RS,12bSR,2′RS)-Ethyl 2′-(1,3,4,6,7,12b-hexahydro-2H-benzo[b]furo[2,3-a]quinolizin-2-yl) propionate Hydrochloride.

Hydrogenation of the Z olefin (150 mg) obtained in Step A according to the procedure described in Step G of Example 1 yielded the free base of the product (100 mg) after chromatography over silica gel, eluting with 20% ethyl acetate/hexane. Conversion of the free base to the HCl salt afforded a white crystalline material, m.p. 251—252°C.

*Step C:* Preparation of (2RS,12bSR,2′SR)-Ethyl 2′-(1,3,4,6,7,12b-hexahydro-2H-benzo[b]furo[2,3-a]quinolizin-2-yl) propionate Hydrochloride.

The E olefin (200 mg) obtained in Step A and 50 mg $PtO_2$ in 50 mL of ethanol was hydrogenated at 40 psi (2.8 × $10^5$ Pa) for 18 hours. The catalyst was filtered, and the solvent was evaporated. The resultant oil was chromatographed over silica gel, eluting with 20% ethyl acetate/hexane to afford the free base of the product. The free base was converted to the HCl salt, m.p. 188—191°C.

Employing the procedure substantially as described in Examples 1 and 2 but substituting for the Wittig reagents and the benzo[b]furoquinolizin-2-ones used therein, the Wittig reagents and arylquinolizin-2-ones described in Table I there are prepared the acetic acid esters also described in Table I, in accordance with the following reaction scheme:

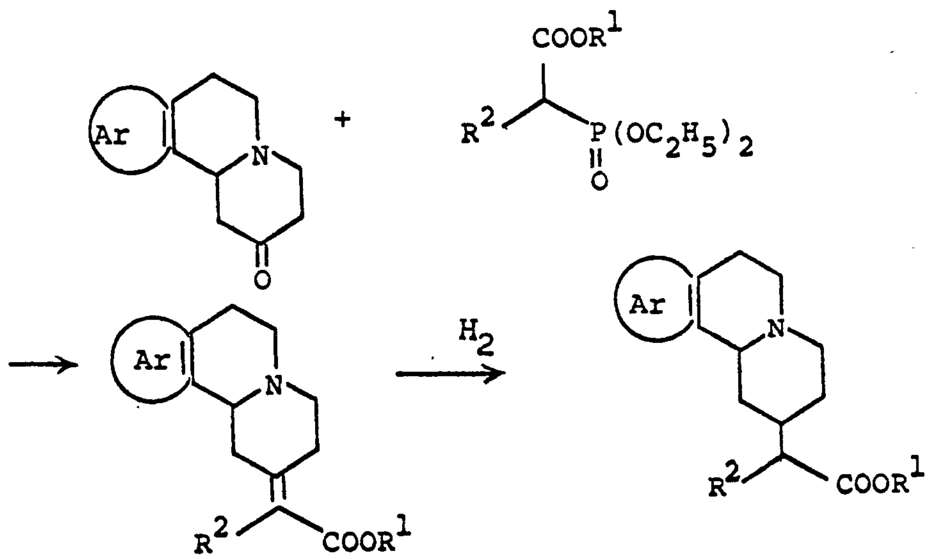

TABLE I

| Ar | $R^2$ | $R^1$ |
|---|---|---|
| benzo- | H | $—C_2H_5$ |
| benzo[b]thieno- | $—CH_3$ | $—CH_3$ |
| thieno- | $—CH(CH_3)_2$ | $—(CH_2)_2CH_3$ |
| furo- | $—CH_2—CH=CH_2$ | $—C_4H_9$ |

Example 3

E and Z 2-(1-Ethylsulfonylethylidene)-1,3,4,6,7,12b-hexahydro-2H-benzo[b]furo[2,3-a]quinolizine Hydrochloride and 2-(1-Ethylsulfonylethyl)-1,6,7,12b-tetrahydro-3H-benzo[b]furo[2,3-a]quinolizine Hydrochloride.

α-Diethylphosphonoethylsulfone (1.34 g; 5.2 mmol) was dissolved in 20 mL of THF and cooled to −70°C under $N_2$ gas. To this solution, n-butyl lithium was added dropwise, and the mixture was stirred for 1 hour. 1,3,4,6,7,12b-Hexahydro-2H-benzo[b]furo[2,3-a]quinolizin-2-one (0.96 g; 4 mmol) in 10 mL of THF was added at −70°C under $N_2$ gas, and the reaction mixture was heated at 50°C overnight. A saturated $NH_4Cl$ solution and ether were added to the reaction mixture, and the ether layer was separated, washed with $H_2O$, dried over $MgSO_4$, and evaporated to give an oil. The free bases of the products were separated by chromatography over silica gel, eluting with 12% ethyl acetate/$CH_2Cl_2$ and converted to HCl salts to give: Z-2-(1-Ethylsulfonylethylidene)-1,3,4,6,7,12b-hexahydro-2H-benzo[b]furo[2,3-a]quinolizine Hydrochloride m.p. 210—212°C; E - 2 - (1 - Ethylsulfonylethylidene) - 1,3,4,6,7,12b - hexahydro - 2H - benzo[b]furo[2,3 - a]quinolizine Hydrochloride m.p. 247—248°C; and 2 - (1 - Ethylsulfonylethyl) - 1,6,7,12b - tetrahydro - 3H - benzo[b]furo[2,3 - a]quinolizine Hydrochloride m.p. 222—223°C.

Example 4

(2RS,12bSR)-Ethyl 1,3,4,6,7,12b-Hexahydro-2H-benzo[b]furo[2,3-a]quinolizine-2-carboxylate Hydrochloride.

*Step A:* Preparation of (12bSR)-2-(1,3-dithian-2-ylidene)-1,3,4,6,7,12b-hexahydrobenzo[b]furo[2,3-a]-quinolizine.

2-Trimethylsilyl-1,3-dithiane (0.77 g; 4 mmol) was dissolved in 10 mL dry THF and cooled to −25°C with a dry ice/$CCl_4$ bath. To this was added 1.6 ml n-butyl lithium (1.6 M solution in hexane) and the solution was stirred for 2 hours at −25°C, after which time 0.422 g (1.75 mmol) (12bRS)-1,3,4,6,7,12b-hexahydro-2H-benzofuro[2,3-a]quinolizin-2-one was added and the reaction mixture was allowed to warm to room temperature (15 minutes). The reaction mixture was poured into 50 mL $H_2O$ and extracted with 3 × 25 mL $CH_2Cl_2$. The combined organics were extracted with 5 × 20 mL $H_2O$, 1 × 25 mL brine, dried ($MgSO_4$) and the solvent was removed *in vacuo.* Purification by medium pressure chromatography ($CHCl_3$) yielded 0.385 g (64%) product as a yellow crystalline solid, m.p. 133—134°C.

*Step B:* Preparation of (2RS,12bSR)-Ethyl 1,3,4,6,7,12b-hexahydro-2H-benzo[b]furo[2,3-a]quinolizine-2-carboxylate Hydrochloride.

(12bSR)-2-(1,3-Dithian-2-ylidenne)-1,3,4,6,7,12b-hexahydrobenzo[b]furo[2,3-a]quinolizine (0.18 g; 0.524 mmol) was dissolved in 30 mL absolute ethanol which previously had been saturated with HCl gas. The reaction was heated to 50°C for 3 hours, the solvent was removed and the residue was partitioned between 25 mL ethyl acetate and 25 mL 20% NaOH solution. The aqueous layer was separated and the organic layer washed with 1 × 20 mL 20% NaOH, 3 × 25 mL $H_2O$, 1 × 25 mL brine, dried ($MgSO_4$) and the solvent was removed *in vacuo.* Purification by spinning disc chromatography (1% methanol/$CHCl_3$) yielded 0.12 g (77%) of the product as an oil. The oil was dissolved in 20 mL ethyl acetate and ethanolic HCl was added to give a white solid, m.p. 228—230°C (dec).

Employing the procedure substantially as described in Example 4, Step B, but substituting for the ethanol reagent used therein comparable amounts of an alcohol of structure $R^1OH$, there are produced the esters described below in accordance with the following reaction scheme:

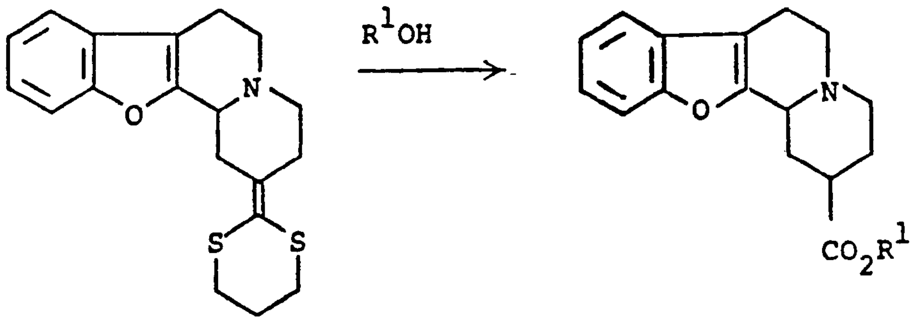


wherein $R^1$ is $-CH_3$, $-CH_2CH_2CH_3$, $-CH(CH_3)_2$, $-CH(CH_3)CH_2CH_3$ and $-(CH_2)_4CH_3$.

Example 5

(2Rs,12bSR)-2-Acetyl-1,3,4,6,7,12b-hexahydro-2H-benzo[b]furo[2,3-a]quinolizine Hydrochloride.

(1-Ethylthio)ethyldiethylphosphonate (2.34 g; 10.4 mmol) was dissolved in 50 mL of THF and cooled in an acetone-dry ice bath under $N_2$ gas. To this solution, n-butyl lithium (8.8 mmol) was added dropwise. After stirring for 3 hours, (12bRS)-1,3,4,6,7,12b-hexahydro-2H-benzo[b]furo[2,3-a]-quinolizin-2-one (1.93 g) 8 mmol) in 20 mL of THF was added to the solution which then was stirred for 1 hour with cooling, for 1 hour at room temperature, and overnight at 50°C. Water (40 mL) and saturated $NH_4Cl$ solution were added successively to the reaction mixture which was then extracted into ether. The ether layer was washed with saturated $NaHCO_3$ solution, brine, and water. Evaporation gave an oil. The oil was dissolved in 20 mL of ethanol and to this solution, 20 mL of ethanol saturated with dry HCl gas and 5 drops of $H_2O$ were added with stirring at room temperature. After stirring overnight, the ethanol was evaporated to give a brown residue which was redissolved in ethyl acetate and made basic with dilute $NH_4OH$. The ethyl acetate layer was separated, washed with $H_2O$, and dried over $K_2CO_3$. Evaporation gave a dark oil which was subjected to silica-gel column chromatography. Ethyl acetate eluted the desired product. The free base was converted to the hydrochloride salt with ethanol/HCl, m.p. >260°C.

Employing the procedure substantially as described in Example 5, but substituting for the Wittig reagent and benzo[b]furoquinolizin-2-one used therein the Wittig reagents and arylquinolizin-2-ones described in Table II, there are produced the 2-alkanoyl-arylquinolizines also described in Table II in accordance with the following reaction scheme:

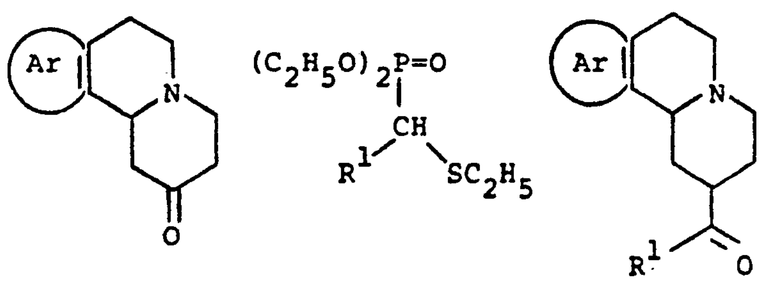

Table II

| Ar | $R^1$ |
|---|---|
| benzo- | $-CH_3$ |
| pyridino- | $-C_3H_7$-n |
| benzo[b]furo- | $-C_2H_5$ |
| benzo[b]thieno- | $-C_4H_9$-n |
| thieno- | $-CH(CH_3)_2$ |
| furo- | $-CH_3$ |

Example 6

(2RS,12bSR)-1,3,4,6,7,12b-Hexahydro-2-(1-hydroxyiminoethyl)-2H-benzo[b]furo[2,3-a]quinolizine Hydrochloride.

Into a mixture of 10 mL of ethanol and 3 mL of pyridine, (2RS,12bSR)-2-Acetyl-1,3,4,6,7,12b-hexahydro-2H-benzo[b]furo[2,3-a]quinolizine (0.4 g; 1.5 mmol) and hydroxylamine hydrochloride (0.1 g; 1.5 mmol) were added. After 2 hours stirring, the product precipitated. The solid was collected by filtration and washed with ethyl acetate several times, m.p. 280°C.

Employing the procedure substantially as described in Example 6, but substituting for the 2-acetyl-benzo[b]furoquinolizine used therein, the 2-alkanoyl-arylquinolizines described in Tables II and III there are produced the oximes described in Table III in accordance with the following reaction scheme:

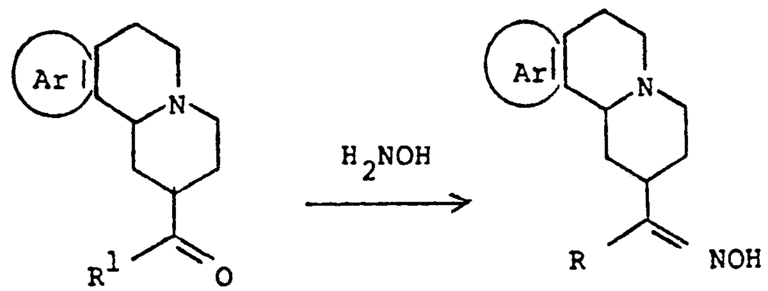


Table III

| Ar | $R^1$ |
|---|---|
| benzo- | $-CH_3$ |
| pyridino- | $-C_3H_7$-n |
| benzo[b]furo- | $-C_2H_5$ |
| benzo[b]thieno- | $-C_4H_9$-n |
| thieno- | $-CH(CH_3)_2$ |
| furo- | $-CH_3$ |

Example 7

(2RS,12bSR)-1,3,4,6,7,12b-Hexahydro-2-(1-methoxyiminoethyl)-2H-benzo[b]furo[2,3-a]quinolizine Hydrochloride.

Methoxyamine hydrochloride (0.17 g; 2.0 mmol) and (2RS,12bSR)-2-Acetyl-1,3,4,6,7,12b-hexahydro-2H-benzo[b]furo[2,3-a]quinolizine (0.45 g; 1.7 mmol) were dissolved in 10 mL of pyridine and stirred at room temperature overnight under $N_2$ gas. The reaction mixture was poured into 3% $NaHCO_3$ solution and then extracted into ethyl acetate. The ethyl acetate layer was washed with brine and $H_2O$, and dried over $K_2CO_3$. Evaporation of the ethyl acetate gave an oil which was subjected to silica gel column chromatography. A mixture of $CH_2Cl_2$ and ethyl acetate (4:1) eluted 280 mg of the free base of the product as an oil.

The oil was transformed into the HCl salt in HCl/ethanol. Evaporation of the ethanol gave a white solid which was recrystallized from ethyl acetate/ether to give the product, m.p. 228°C (d).

Example 8

(2RS,12bSR)-1,3,4,6,7,12b-Hexahydro-2-(1-hydroxyethyl)-2H-benzo[b]furo[2,3-a]quinolizine Hydrochloride.

Into an ether solution of (2RS,12bSR)-2-acetyl-1,3,4,6,7,12b-hexahydro-2H-benzo[b]furo[2,3-a]-quinolizine (1.4 g), $LiAlH_4$ (0.4 g) was added and the mixture was stirred under $N_2$ overnight. Water, 15% NaOH, and water were added successively. A precipitated solid was filtered off and from the mother liquor the ether layer was separated which was then washed with water, dried over $MgSO_4$, and evaporated to give the crude alcohol product. The alcohol was subjected to column chromatography and transformed into the HCl salt, recrystallized from methanol/ethyl acetate/ether, to yield 400 mg, m.p. 235°C (d).

Employing the procedure substantially as described in Example 8 but substituting for the 2-acetyl-benzo[b]furoquinolizine used therein, the 2-alkanoyl-arylquinolizines described in Tables II and IV, there are

produced the 1-hydroxyalkyl derivatives described in Table IV in accordance with the following reaction scheme:

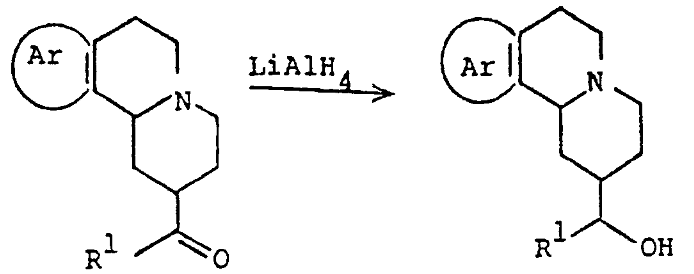


Table IV

| Ar | $R^1$ |
|---|---|
| benzo- | $-CH_3$ |
| pyridino- | $-C_3H_7$-n |
| benzo[b]furo- | $-C_2H_5$ |
| benzo[b]thieno- | $-C_4H_9$-n |
| thieno- | $-CH(CH_3)_2$ |
| furo- | $-CH_3$ |

Example 9

(2RS,12bSR)-1,3,4,6,7,12b-Hexahydro-2-(1-methyl-1-hydroxyethyl) 2H-benzo[b]furo[2,3-a]quinolizine Hydrochloride.

Methylmagnesium bromide (1 mmol) was added to a solution of (2RS,12bSR)-2-acetyl-1,3,4,6,7,12b-hexahydro-2H-benzo[b]furo[2,3-a]quinolizine (0.269 g; 1 mmol) in 15 mL ether at 0°C. After 2 hours 20 mL of saturated $NH_4Cl$ solution was added to the reaction mixture. The organic layer was separated and evaporated to a yellow solid which was crystallized from ether. The free base was converted to the hydrochloride salt by treatment with ethanolic HCl to give the product, m.p. 255—256°C.

Employing the procedure substantially as described in Example 9, but substituting for the methyl magnesium bromide and the 2-acetyl-benzo[b]furoquinolizine used therein, the Grignard reagents and 2-alkanoyl-arylquinolizines described in Table V, there are produced the tertiary alcohols also described in Table V in accordance with the following reaction scheme:

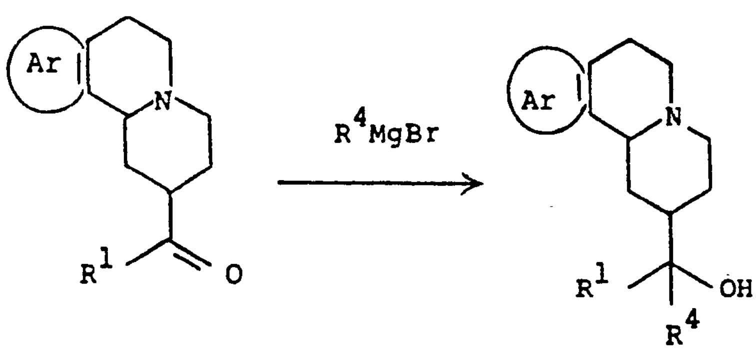


Table V

| Ar | $R^1$ | $R^4$ |
|---|---|---|
| benzo- | $-CH_3$ | $-CH_3$ |
| pyridino- | $-C_3H_7$-n | $-C_2H_5$ |
| benzo[b]furo- | $-C_2H_5$ | $-C_3H_7$-n |
| benzo[b]thieno- | $-C_4H_9$-n | $-CH_3$ |
| thieno- | $-CH(CH_3)_2$ | $-CH_3$ |
| furo- | $-CH_3$ | $-CH_2-CH=CH_2$ |

Example 10

(2RS,12bSR)-1,3,4,6,7,12b-Hexahydro-2-(1-(2-hydroxyethoxy)ethyl) 2H-benzo[b]furo[2,3-a]quinolizine Hydrochloride.

*Step A:* Preparation of (2RS,12bSR) Ethyl 1-(1,3,4,6,7,12b-hexahydro-2H-benzo[b]furo[2,3-a]quinolizin)2-yl)ethoxyacetate Hydrochloride.

A solution of (2RS,12bSR)-1,3,4,6,7,12b-hexahydro-2-(1-hydroxyethyl)-2H-benzo[b]furo[2,3-a]-quinolizine (0.7 g; 2.7 mmol) in 20 mL of DMF-benzene (1:1) was added to a suspension of NaH (5.4 mmol) in 20 mL of DMF-benzene at 0°C with stirring. After the subsequent addition of ethyl bromoacetate (0.45 g; 3 mmol), the reaction was stirred at 25°C for 3 hours. Dilute HCl was added until the reaction mixture was acidic and then $NH_4OH$ was added until the mixture was basic. The mixture was extracted with ether. The organic extracts were washed with water, dried over $MgSO_4$, and evaporated to give the product as an oil.

*Step B:* Preparation of (2RS,12bSR)-1,3,4,6,7,12b-hexahydro-2-(1-(2-hydroxyethoxy)ethyl)-2H-benzo[b]furo[2,3-a]quinolizine Hydrochloride.

A solution of the ester obtained in Step A above (0.32 g) and $NaBH_4$ (0.087 g) in 4 mL of t-butanol was heated to reflux. Methanol (0.8 mL) was added over 1 hour. The reaction mixture was cooled and water was added. The solvent was evaporated, and the residue was extracted with ether. The ether extracts were washed with water, dried over $MgSO_4$, and evaporated to give the free base of the product as an oil. The oil was converted to the hydrochloride salt, m.p. 147°C (dec).

Employing the procedure substantially as described in Example 10, but substituting for the 2-(1-hydroxyethyl)-benzo[b]furoquinolizine used therein, the 2-(1-hydroxyalkyl)-arylquinolizines described in Table VI, there are produced the hydroxyethyl ethers also described in Table VI in accordance with the following reaction scheme:

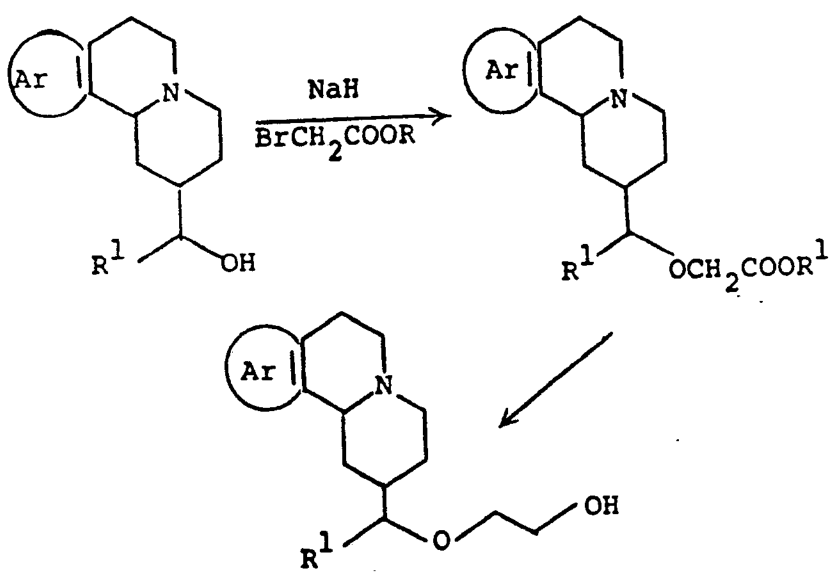


Table VI

| Ar | $R^1$ |
|---|---|
| benzo- | $-CH_3$ |
| pyridino- | $-C_3H_7$-n |
| benzo[b]furo- | $-C_2H_5$ |
| benzo[b]thieno- | $-C_4H_9$-n |
| thieno- | $-CH(CH_3)_2$ |
| furo- | $-CH_3$ |

Example 11

(2RS,12bSR)-1,3,4,6,7,12b-Hexahydro-2-(1-(2-hydroxyethylamino)ethyl) 2H-benzo[b]furo[2,3-a]quinolizine Dihydrochloride.

A solution of ethanolamine (0.136 g), $NaCNBH_3$ (0.46 g), 5N methanolic HCl (0.15 mL), and (2RS,12bSR)-2-acetyl-1,3,4,6,7,12b-hexahydro-2H-benzo[b]furo[2,3-a]quinolizine (0.1 g) in 2 mL of methanol was stirred at 50°C for 18 hours. The reaction mixture was first made acidic with dilute HCl and then basic with $NH_4OH$. The resulting mixture was extracted with $CHCl_3$. The extracts were washed with water, dried over $MgSO_4$, and evaporated to give the free base of the product as an oil. The oil was converted to the dihydrochloride salt and recrystallized from methanol-ether to give the product, m.p. 250°C.

Example 12

(2RS,12bSR)-1,3,4,6,7,12b-Hexahydro-2-(1-methyl-1-(2-hydroxyethoxy)ethyl)-2H-benzo[b]furo[2,3-a]quinolizine Hydrochloride.

*Step A:* Preparation of (2RS,12bSR)-1,3,4,6,7,12b-hexahydro-2-(2-methyl)1,3-dioxalan-2-yl)-2H-benzo[b]furo[2,3-a]quinolizine.

A mixture of ethyleneglycol (1 mL), borontrifluoride etherate (1 mL), and (2RS,12bSR)-2-acetyl-1,3,4,6,7,12b-hexahydro-2H-benzo[b]furo[2,3-a]quinolizine (0.5 g) in 2 mL of $CH_2Cl_2$ was stirred at 25°C. for 18 hours in the dark. Water (10 mL) was then added with vigorous stirring. The methylene chloride was evaporated, and the residual aqueous mixture was extracted with ethyl acetate. The organic extract was washed with water, dried over $MgSO_4$, and evaporated. Chromatography over silica gel, eluting with methylene chloride-ethyl acetate, afforded the product as an oil.

*Step B:* Preparation of (2RS,12bSR)-1,3,4,6,7,12b-hexahydro-2-(1-methyl-1-(2-hydroxyethoxy)ethyl)-2H-benzo[b]furo[2,3-a]quinolizine Hydrochloride.

The oil (0.4 g) from Step A and methyl-magnesium bromide (12.7 mmol) were dissolved in benzene, and the mixture was refluxed for 18 hours. Into the cooled reaction mixture, 20 mL of 25% ammonium acetate was added, and the benzene layer was separated. The organic extracts were washed with water, dried over $MgSO_4$, and evaporated to give an oil. The oil was chromatographed over silica gel, eluting with $CH_2Cl_2$/ethyl acetate/ethanol (4:2:1), to afford the free base of the product which was converted into the hydrochloride salt, m.p. 222—223°C.

Employing the procedure substantially as described in Example 12, but substituting for the 2-acetyl-benzo[b]furoquinolizine and methyl magnesium bromide used therein, the 2-alkanoyl-aryquinolizines and the Grignard reagents described in Table VII, there are produced the hydroxyethoxy compounds, also described in Table VII in accordance with the following reaction scheme:

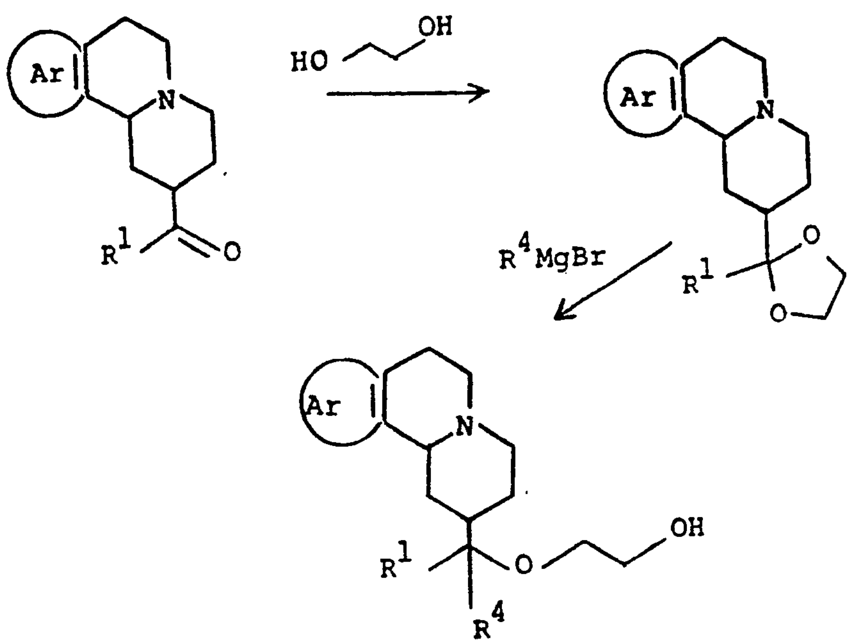

Table VII

| Ar | $R^1$ | $R^4$ |
|---|---|---|
| benzo- | $-CH_3$ | $-CH_3$ |
| pyridino- | $-C_3H_7$-n | $-C_2H_5$ |
| benzo[b]furo- | $-C_2H_5$ | $-C_3H_7$-n |
| benzo[b]thieno- | $-C_4H_9$-n | $-CH(CH_3)_2$ |
| thieno- | $-CH(CH_3)_2$ | $-CH_2-CH=CH_2$ |
| furo- | $-CH_3$ | $-CH_3$ |

Example 13

(2RS,12bSR)-1,3,4,6,7,12b-Hexahydro-2-(1-(2 hydroxyethylthio)ethenyl)-2H-benzo[b]furo[2,3-a]quinolizine.

*Step A:* Preparation of (2RS,12bSR)-1,3,4,6,7,12b-hexahydro-2-(2-methyl-1,3-oxathiolan-2-yl)-2H-benzo[b]furo[2,3-a]quinolizine.

A solution of (2RS,12bSR)-2-acetyl-1,3,4,6,7,12b-hexahydro 2H-benzo[b]furo[2,3-a]quinolizine (0.25 g), 2-mercaptoethanol (0.2 mL), and borontrifluoride etherate (0.4 mL) in 5 mL of acetic acid was stirred at 25°C. for 18 hours. The acetic acid was evaporated, and $NH_4OH$ was added to the residue. The aqueous mixture was extracted with ethyl acetate. Evaporation of the organic extracts afforded an oil which was chromatographed over silica gel, eluting with methylene chloride-ethyl acetate (1:1) to give 0.25 g of the product as an oil.

*Step B:* Preparation of (2RS,12bSR)-1,3,4,6,7,12b-hexahydro-2-(1-(2-hydroxyethylthio)ethenyl)-2H-benzo[b]furo[2,3-a]quinolizine.

Aluminum chloride (0.116 g) was placed in a flask and cooled in an ice bath. Cold ether (2 mL) was added, and the mixture stirred 15 minutes until solution was affected. $LiAlH_4$ (0.01 g) was added in 1 mL of ether. Fifteen minutes later, the product from Step A (0.23 g) in 2 mL of ether was added, and the ice bath was removed. The reaction mixture was heated at reflux for 2 hours. Water (0.2 mL) was added, followed by 1 mL of 10% $H_2SO_4$. A saturated solution of $NaHCO_3$ was then added until the reaction was basic. The reaction mixture was extracted with ethyl acetate. The extracts were washed with water, dried over $MgSO_4$, and evaporated to afford an oil. The oil was chromatographed over silica gel and then recrystallized to yield 0.022 g of the product, m.p. 116—117°C.

Example 14

(2RS,12bSR)-N-1,3,4,6,7,12b-Hexahydro-2H-benzo[b]furo[2,3-a]quinolizin-2-yl)methyl-N-methyl-N'-dimethylsulfamide Hydrochloride.

*Step A:* Preparation of (2RS,12bSR)-1,3,4,6,7,12b-hexahydro-2-methoxymethylidene-2H-benzo[b]furo[2,3-a]quinolizine.

To a cold (0°C) solution of diisopropylamine (0.22 g; 2.2 mmole) in 6 mL of tetrahydrofuran (THF) was added n-butyl lithium (1 mL of a 2.1 molar solution, 2.1 mmole). After 15 minutes this mixture was added via syringe to a suspension of methoxymethyltriphenylphosphine chloride (0.685 g; 2.0 mmole) in 5 mL of toluene at 0°C, stirred 15 minutes, and then a solution of (12bRS)-1,3,4,6,7,12b-hexahydro-2H-benzo[b]furo[2,3-a]quinolizin-2-one (0.12 g; 0.5 mmole) in 3 mL of toluene was added. After 30 minutes, the reaction mixture was poured into water (50 mL), washed with ether (2 × 40 mL), the ether washed with brine (40 mL), dried ($Na_2SO_4$), filtered and concentrated. The oily residue was chromatographed (silica, ethyl acetate/hexane, 1:1) to give 104 mg of a 1:1 mixture of E and Z enol ethers as an oil. Exact mass-calc 269.1416, Found 269.14163.

*Step B:* Preparation of (2Rs,12bSR) and (2SR,12bSR)-1,3,4,6,7,12b-Hexahydro-2H-benzo[b]furo[2,3-a]-quinolizine-2-carboxaldehyde.

The mixture of enol ethers (104 mg) from Step A was dissolved in 6 mL of 2N HCl, stirred for 4 hours at 25°C, made basic and extracted with methylene chloride (2 × 40 mL), dried ($Na_2SO_4$), filtered and concentrated to give 60 mg of crude aldehyde a (2:1) mixture of (2RS,12bSR):(2SR,12bSR) aldehydes.

*Step C:* Preparation of (2Rs,12bSR)-1,3,4,6,7,12b-Hexahydro-2-methylaminomethyl-2H-benzo[b]furo[2,3-a]quinolizine.

To a solution of aldehydes from Step B (0.15 g; 0.56 mmole) in 3 mL of methanol was added methylamine hydrochloride (0.18 g; 2.8 mmol) and sodium cyanoborohydride (0.050 g; 0.78 mmole). After 48 hours at 25°C, the reaction was concentrated to dryness, 5 mL of 6N HCl added, stirred 30 minutes, diluted with 30 mL of water, made basic and extracted with methylene chloride (2 × 30 mL), dried over

$Na_2SO_4$), filtered and concentrated to an oil which was chromatographed (silica, $NH_3$ saturated $CHCl_3$/1% MeOH) to give 65 mg of product.

*Step D:* Preparation of (2Rs,12bSR)-N-(1,3,4,6,7,12b-Hexahydro-2H-benzo[b]furo[2,3-a]quinolizin-2-yl)methyl-N-methyl-n′-dimethyl sulfamide Hydrochloride.

To a solution of the amine from Step C (0.065 g; 0.24 mmole) in 2 mL of methylene chloride was added 0.050 g (0.48 mmole) of triethylamine and 0.070 g (0.48 mmole) of dimethylsulfamoyl chloride. After 18 hours at 25°C, this was poured into 10 mL of saturated $NaHCO_3$, extracted into methylene chloride (2 × 20 mL), dried ($Na_2SO_4$), filtered and concentrated to give an oil which was chromatographed (silica, ethyl acetate) to give 0.05 mg of the free base of the product. The HCl.1/4$H_2O$ salt was generated in ethyl acetate and ethanolic HCl, m.p. 242—245°C.

Example 15

Pharmaceutical Formulation

| Ingredient | Mg/Capsule |
|---|---|
| (2RS,12bSR)-Methyl (1,3,4,6,7,12b-hexahydro-2H-benzo[b]furo[2,3-a]-quinolizin-2-yl) acetate Hydrochloride | 6 |
| starch | 87 |
| magnesium stearate | 7 |

The active ingredient, starch and magnesium stearate are blended together. The mixture is used to fill hard shell capsules of a suitable size at a fill weight of 100 mg per capsule.

Example 16

| Ingredient | Mg/Capsule |
|---|---|
| (2RS,12bSR)-1,3,4,6,7,12b-Hexahydro-2-(1-methyl-1-hydroxyethyl)-2H-benzo[b]furo[2,3-a]quinolizine Hydrochloride | 6 |
| starch | 87 |
| magnesium stearate | 7 |

**Claims**

1. Compounds of structural formula I

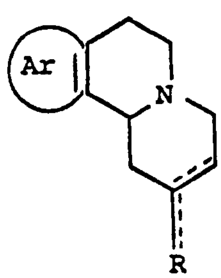

I

or salts of the compounds of formula I wherein Ar represents an aromatic or heteroaromatic group selected from

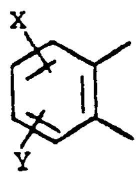 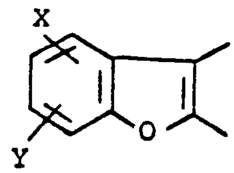 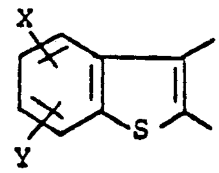

benzo- benzofuro- benzothieno-

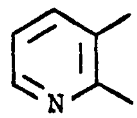 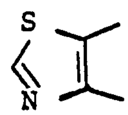 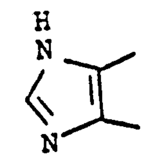 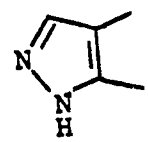

pyridino- thiazolo- imidazo- pyrazolo-

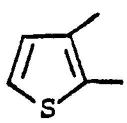 and 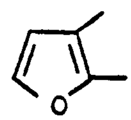

thieno- furo-

wherein
X and Y are independently:
1) hydrogen,
2) halo,
3) hydroxy,
4) $C_{1-3}$alkoxy, or
5) $C_{1-6}$alkyl;
R is
1) $COOR^1$ wherein
$R^1$ is hydrogen or $C_{1-5}$alkyl, either straight or branched chain;

2) 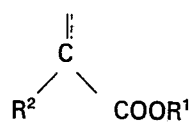

wherein
$R^1$ is as defined in (1) and
$R^2$ is a) hydrogen,
b) $C_{1-5}$alkyl, or
c) allyl

3) 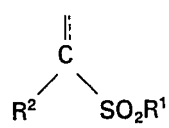

wherein $R^1$ and $R^2$ are as defined in (2)

4) 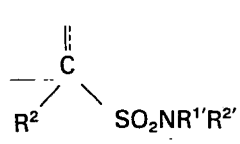

wherein
$R^{1'}$ and $R^{2'}$ have the same meaning as $R^1$ and $R^2$ in (2) or
$R^{1'}$ and $R^{2'}$ form together with the nitrogen atom to which they are bonded a 5- or 6-membered

heterocycle which is selected from the group comprising pyrrolidino, morpholino, piperidino, and piperazino;

5) 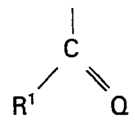

wherein

Q is oxygen or a group $=N—OR^1$ wherein $R^1$ has the same meaning as in (1)

6) 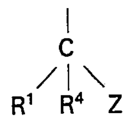

wherein

Z is a) $—OR^3$ or $—SR^3$

wherein

$R^3$ is

i) is

ii) $C_{1-5}$alkyl, unsubstituted or substituted with

1) —OH,

2) $—COOR^1$,

3) $—SO_2R^2$,

4) $—N(R^2)SO_2R^2$,

wherein

$R^1$ and $R^2$ are as defined in (2) or b) $—NR^2R^3$

wherein

$R^2$ is as defined in (2) and

$R^3$ is as defined in (a) above

$R^4$ is a) hydrogen

b) $C_{1-5}$alkyl, or

c) $C_{1-5}$alkylidene or allyl and

$R^1$ is as defined in (2) and the broken lines represent possible double bonds.

2. Compounds according to claim 1 characterized in that in formula I Ar is X,Y-benzo, X,Y-benzo[b]furo- or X,Y-benzo[b]thieno- or salts of said compounds.

3. Compounds according to claim 2 characterized in that Ar is benzo[b] furo-.

4. Compounds according to one of the claims 1—3, characterized in that in formula I R is one of the following groups

$$—CH_2—CO_2R^1,\quad CH_3\overset{|}{C}HCO_2R^1,\quad R^1—\overset{|}{C}=NOR^1,\quad R^1(R^4)\overset{|}{C}—OH,$$

$$R^1—\overset{|}{C}H—O(CH_2)_2OH,\ \text{or}\quad R^1(R^4)\overset{|}{C}—O—(CH_2)_2OH$$

wherein

$R^1$ and $R^4$ are independently hydrogen atoms or alkyl groups having 1—5 carbon atoms or salts of said compounds.

5. Compounds according to claim 4 characterized in that in the groups of the stated formulae $R^1$ and $R^4$ are hydrogen or methyl, preferably methyl, or salts of said compounds.

6. Process for the preparation of compounds of formula Ia

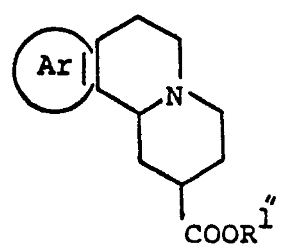

Ia

or salts thereof wherein

Ar is as defined in formula I of claim 1 and $R^{1''}$ is $C_{1-5}$alkyl characterized in that a compound of formula II

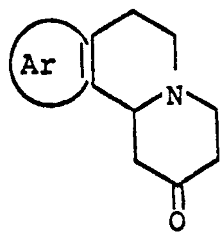

II

is reacted with 2-trimethylsilyl-1,3-dithiane of formula III

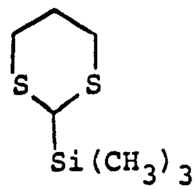

III

in the presence of an etheral solvent and a strong base yielding a 1,3-dithian-2-ylidene derivative of formula IV

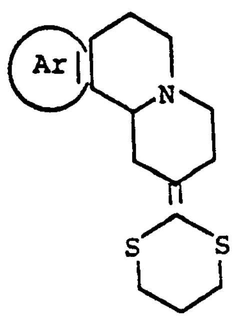

IV

which is reacted with a $C_{1-5}$ alkanol of formula $R^{1''}$——OH, which alkanol is saturated with hydrogen chloride yielding the desired final products of formula Ia, which are isolated in the free form or as salts thereof.

7. Process for the preparation of compounds of formula I according to claim 1 characterized in that compounds of formula Ib

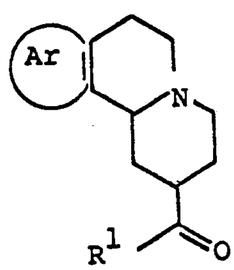

Ib

or salts thereof are prepared wherein Ar and $R^1$ have the same meaning as in claim 1 characterized in that a compound of formula II

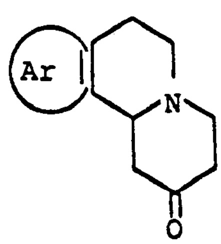

II

is reacted with a phosphorous compound of formula

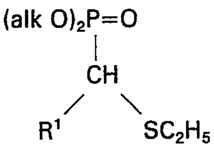

wherein

$R^1$ is hydrogen or $C_{1-5}$ alkyl, or the compounds of formula Ib are prepared, wherein $R^1$ is hydrogen by reacting the compound of formula II with a phosphorus compound of formula

$$Ph_3P^+—CH_2—O—CH_3Cl^-,$$

and wherein the intermediate products produced through the reaction with said phosphorus compounds are hydrolized to yield the compounds of formula Ib, and wherein the hydrolization is performed with diluted mineral acid, if the used phosphorus compound is the triphenylphosphoro compound and the hydrolization is performed with saturated aqueous ammonium chloride provided that the used phosphorus compound is an (1-ethylthio) alkyl dialkyl phosphonate and wherein the compounds of formula Ib are isolated in the free form or as salts thereof and which compounds of formula Ib are optionally further reacted with a Grignard reagent of formula $R^{4'}$ MgHal in which $R^{4'}$ is $C_{1-5}$ alkyl, $C_{1-5}$ alkylidene or allyl and

Hal is haline
to yield the compounds of formula Ic

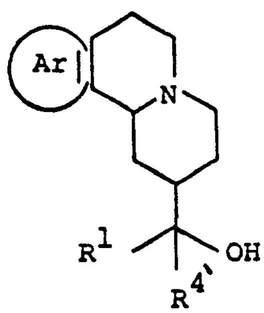

Ic

which compounds of formula Ic are isolated in the free form or as salts thereof or that optionally the keto-compounds of formula Ib are further reacted with a hydroxylamine or alkoxyamine of formula

$$H_2NOR^1$$

in the presence of an organic base to yield the compounds of formula Id

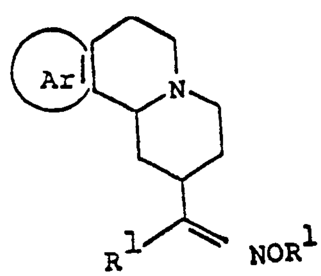

Id

which compounds of formula Id are isolated in the free form or as salts thereof or that optionally the keto-compounds of formula Ib are thereafter reduced, preferably with a complex metal hydride to yield the compounds of formula Ie

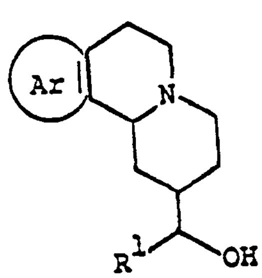

Ie

which compounds of formula Ie are isolated in the free form or as salts thereof or that optionally the keto-compounds of formula Ib are further reductively aminated with an amine of formula

$$R^2\text{—NH—}R^3$$

wherein $R^2$ is hydrogen and $R^3$ is $C_{1-5}$ alkyl or hydroxyethyl, which reductive amination is preferably performed in the presence of an cyano-boro-hydride and methanol containing hydrogen chloride, and the corresponding amino compounds are isolated in the free form or as salts thereof.

8. Process for the preparation of compounds of formula If

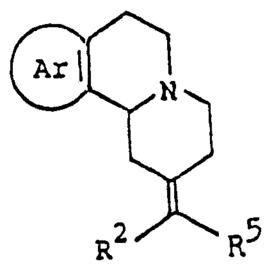

If

according to claim 1 or salts thereof
wherein
$R^5$ is $—COOR^1$
$—SO_2R^1$ or
$—SO_2NR^{1'}R^{2'}$
wherein
Ar, $R^1$, $R^2$, $R^{1'}$ and $R^{2'}$ have the same meaning as in claim 1 characterized in that a compound of formula II

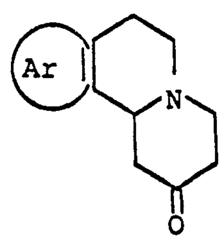

II

is reacted with a Wittig reagent, preferably of formula

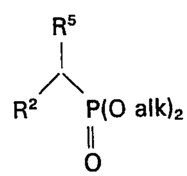

in an inert aprotic solvent in the presence of a strong base to yield the compound of formula If which are isolated as free compounds or as salts thereof and wherein the compound of formula If are optionally further reduced, preferably by hydrogenation in the presence of a noble metal catalyst, to yield the compound of Ig

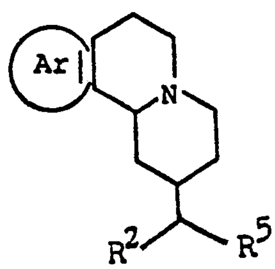

Ig

which are isolated in the free form or as salts thereof.

9. Pharmaceutical composition for the treatment of depression, hypertension, ocular hypertension, diabetes, obesity or platelet aggregation or for modifying gastrointestinal motility, characterized in that it

comprises as pharmaceutically effective ingredient a compound of formula I according to claim 1 or a pharmaceutically acceptable salt thereof.

10. Pharmaceutical composition according to claim 9, characterized in that it comprises as active ingredient a compound according to one of the claims 2—5 or a pharmaceutically acceptable salt thereof and optionally furthermore a pharmaceutical carrier material.

**Patentansprüche**

1. Verbindungen der Strukturformel I

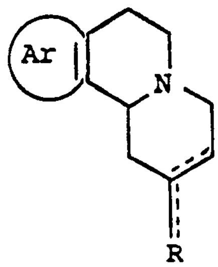

I

oder Salze der Verbindungen der Formel I, worin Ar eine aromatische oder heteroaromatische Gruppe darstellt, ausgewählt aus

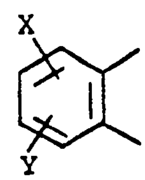

Benzo-

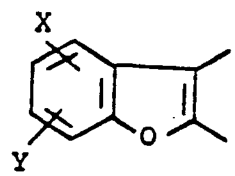

Benzofuoro-

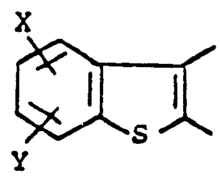

Benzothieno-

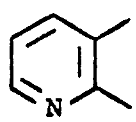

Pyridino-

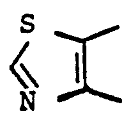

Thiazolo-

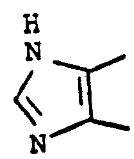

Imidazo-

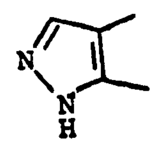

Pyrazolo-

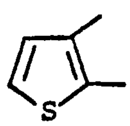

Thieno-

und

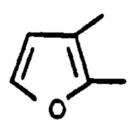

Furo-

worin X und Y unabhängig
1) Wasserstoff,
2) Halogen,
3) Hydroxy,
4) $C_{1-3}$-Alkoxy oder
5) $C_{1-6}$-Alkyl sind;

R 1) $COOR^1$, worin $R^1$ Wasserstoff oder $C_{1-5}$-Alkyl, entweder geradkettig oder verzweigt, ist;

2)

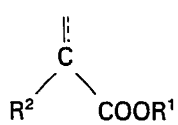

worin $R^1$ wie in (1) definiert ist und
$R^2$ a) Wasserstoff,
b) $C_{1-5}$-Alkyl oder
c) Allyl ist,

3) 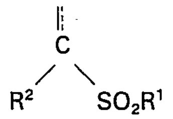

worin $R^1$ und $R^2$ wie in (2) definiert sind,

4) 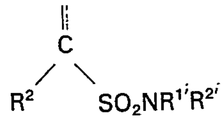

worin $R^{1'}$ und $R^{2'}$ die gleiche Bedeutung haben wie $R^1$ und $R^2$ in (2) oder
$R^{1'}$ und $R^{2'}$ zusammen mit dem Stickstoff, an den sie gebunden sind, einen 5- oder 6gliedrigen Hetero cyclus bilden, der aus der Pyrrolidino, Morpholino, Piperidino und Piperazino umfassenden Gruppe ausgewählt ist;

5) 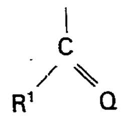

worin Q Sauerstoff oder eine Gruppe $=N-OR^1$ ist, worin $R^1$ die gleiche Bedeutung hat wie in (1),

6) 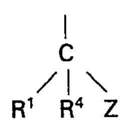 ist,

worin Z
a) $-OR^3$ oder $-SR^3$ ist,
worin $R^3$
i) H,
ii) $C_{1-5}$-Alkyl, unsubstituiert oder substituiert mit
1) $-OH$,
2) $-COOR^1$,
3) $-SO_2R^2$,
4) $-N(R^2)SO_2R^2$ ist,
worin $R^1$ und $R^2$ wie in (2) definiert sind, oder
b) $-NR^2R^3$ ist,
worin $R^2$ wie in (2) definiert ist und $R^3$ wie in (a) oben definiert ist,
$R^4$ a) Wasserstoff,
b) $C_{1-5}$-Alkyl oder
c) $C_{1-5}$-Alkyliden oder Allyl ist und
$R^1$ wie in (2) definiert ist und die gestrichelten Linien mögliche Doppelbindungen darstellen.

2. Verbindungen nach Anspruch 1, dadurch gekennzeichnet, daß in Formel I Ar X,Y-Benzo, X,Y-Benzo[b]furo- oder X,Y-Benzo[b]thieno- ist oder Salze dieser Verbindungen.

3. Verbindungen nach Anspruch 2, dadurch gekennzeichnet, daß Ar Benzo[b]furo- ist.

4. Verbindungen nach einem der Ansprüche 1 bis 3, dadurch gekennzeichnet, daß in Formel I R eine der folgenden Gruppen ist

$-CH_2-CO_2R^1$, $CH_3CHCO_2R^1$, $R^1-C=NOR^1$, $R^1(R^4)C-OH$,

$R^1-CH-O(CH_2)_2OH$, oder $R^1(R^4)C-O-(CH_2)_2OH$

worin $R^1$ und $R^4$ unabhängig Wasserstoffatome oder Alkylgruppen mit 1 bis 5 Kohlenstoffatomen sind, oder Salze dieser Verbindungen.

5. Verbindungen nach Anspruch 4, dadurch gekennzeichnet, daß in den Gruppen der aufgeführten Formeln $R^1$ und $R^4$ Wasserstoff oder Methyl, vorzugsweise Methyl, sind, oder Salze dieser Verbindungen.

6. Verfahren zur Herstellung von Verbindungen der Formel Ia

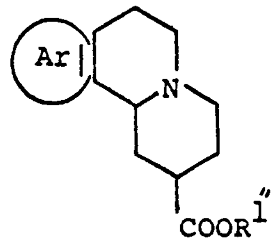

Ia

oder von Salzen davon, worin Ar wie in Formel I von Anspruch 1 definiert ist und $R^{1''}$ $C_{1-5}$-Alkyl ist, dadurch gekennzeichnet, daß eine Verbindung der Formel II

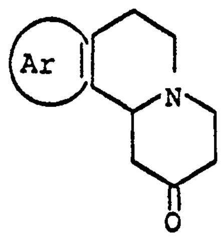

II

mit 2-Trimethylsilyl-1,3-dithian der Formel III

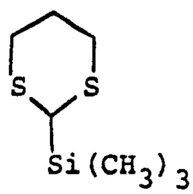

III

in Gegenwart eines etherischen Lösungsmittels und einer starken Base unter Erhalt eines 1,3-Dithian-2-ylidenderivats der Formel IV umgesetzt wird

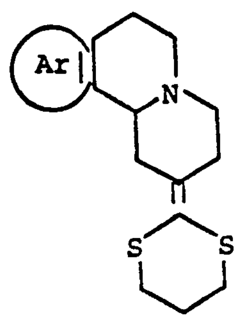

IV

die mit einem $C_{1-5}$-Alkanol der Formel $R^{1''}$—OH umgesetzt wird, wobei das Alkanol mit Chlorwasserstoff gesättigt ist, was die erwünschten Endprodukte der Formel Ia ergibt, die in freier Form oder als Salz davon isoliert werden.

7. Verfahren zur Herstellung von Verbindungen der Formel I nach Anspruch 1, dadurch gekennzeichnet, daß Verbindungen der Formel Ib

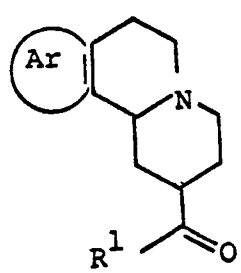

Ib

oder Salze davon hergestellt werden, worin Ar und $R^1$ die gleiche Bedeutung haben, wie in Anspruch 1, dadurch gekennzeichnet, daß eine Verbindung der Formel II

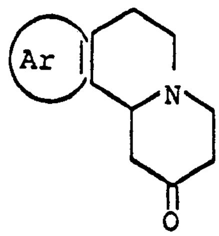

II

mit einer Phosphorverbindung der Formel

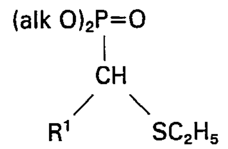

umgesetzt wird, worin $R^1$ Wasserstoff oder $C_{1-5}$-Alkyl ist, oder Verbindungen der Formel Ib hergestellt werden, worin $R^1$ Wasserstoff ist, durch Umsetzung der Verbindung der Formel II mit einer Phosphorverbindung der Formel

$$Ph_3P^+{-}CH_2{-}O{-}CH_3Cl^-$$

und worin die durch die Reaktion mit diesen Phosphorverbindungen erzeugten Zwischenprodukte unter Erhalt der Verbindungen der Formel Ib hydrolisiert werden, und worin die Hydrolysierung mit verdünnter Mineralsäure durchgeführt wird, falls die verwandte Phosphorverbindung eine Triphenylphosphoroverbindung ist, und die Hydrolysierung mit gesättigtem wäßrigem Ammoniumchlorid durchgeführt, wenn die verwandte Phosphorverbindung ein (1-Ethylthio)alkyldialkylphosphonat ist, und worin die Verbindungen der Formel Ib in der freien Form oder als ihre Salze isoliert werden, und wobei die Verbindungen der Formel Ib gegebenenfalls mit einem Grignard-Reagenz der Formel $R^{4'}MgHal$ weiter umgesetzt werden, worin $R^{4'}$ ein $C_{1-5}$-Alkyl, $C_{1-5}$-Alkyliden oder Allyl ist und Hal Halogen ist, wobei Verbindungen der Formel Ic

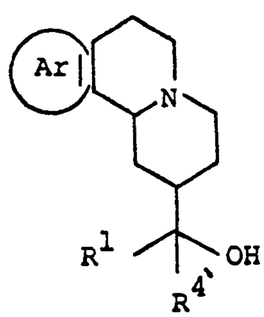

Ic

erhalten werden, welche Verbindungen der Formel Ic in freier Form oder als Salze davon isoliert werden, oder daß gegebenenfalls die Ketoverbindungen der Formel Ib mit einem Hydroxylamin oder Alkoxyamin der Formel

$$H_2NOR^1$$

in Gegenwart einer organischen Base unter Erhalt der Verbindungen der Formel Id umgesetzt werden

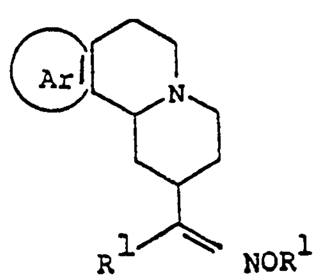

Id

welche Verbindungen der Formel Id in freier Form oder als Salze davon isoliert werden, oder daß gegebenenfalls die Ketoverbindungen der Formel Ib danach reduziert werden, vorzugsweise mit einem komplexen Metallhydrid, unter Erhalt der Verbindungen der Formel Ie

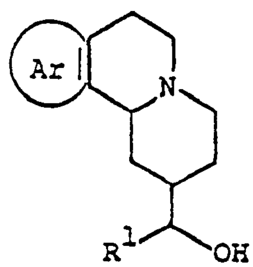

Ie

welche Verbindungen der Formel Ie in freier Form oder als Salze davon isoliert werden, oder daß gegebenenfalls die Ketoverbindungen der Formel Ib weiter mit einem Amin der Formel

$$R^2—NH—R^3$$

reduktiv aminiert werden, worin $R^2$ Wasserstoff ist und $R^3$ $C_{1-5}$-Alkyl oder Hydroxyethyl ist, welche reduktive Aminierung vorzugsweise in Gegenwart eines Cyanoborhydrids und von chlorwasserstoffhaltigem Methanol durchgeführt wird, wobei die entsprechenden Aminoverbindungen in freier Form oder als Salze davon isoliert werden.

8. Verfahren zur Herstellung von Verbindungen der Formel If

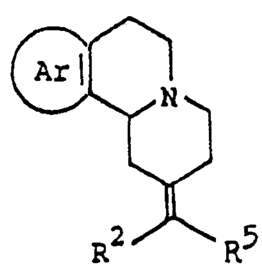

If

nach Anspruch 1 oder Salzen davon, worin $R^5$
$—COOR^1$,
$—SO_2R^1$ oder
$—SO_2NR^{1'}R^{2'}$ ist,
worin Ar, $R^1$, $R^2$, $R^{1'}$ und $R^{2'}$ die gleiche Bedeutung haben wie in Anspruch 1, dadurch gekennzeichnet, daß eine Verbindung der Formel II

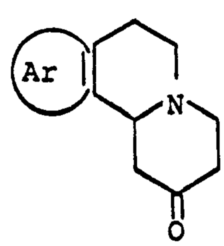

II

mit einem Wittig-Reagenz vorzugsweise der Formel

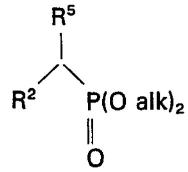

in einem inerten aprotischen Lösungsmittel in Gegenwart einer starken Base unter Erhalt der Verbindungen der Formel If umgesetzt wird, die als freie Verbindungen oder als Salze davon isoliert werden, und worin die Verbindungen der Formel If gegebenenfalls weiter reduziert werden, vorzugsweise

durch Hydrierung in Gegenwart eines Edelmetallkatalysators, wobei die Verbindungen Ig erhalten werden

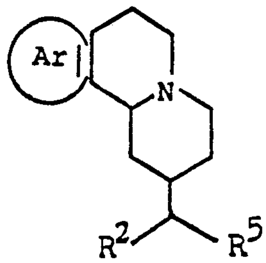

Ig

die in freier Form oder als Salze davon isoliert werden.

9. Pharmazeutische Zusammensetzung zur Behandlung von Depressionen, Bluthochdruck, Augenüberdruck, Diabetes, Fettsucht oder Blutplättchenaggregationen, oder zur Modifizierung gastrointestinaler Motilität, dadurch gekennzeichnet, daß sie als pharmazeutisch wirksamen Bestandteil eine Verbindung der Formel I gemäß Anspruch 1 oder ein pharmazeutisch annehmbares Salz davon umfaßt.

10. Pharmazeutische Zusammensetzung nach Anspruch 9, dadurch gekennzeichnet, daß sie als aktiven Bestandteil eine Verbindung nach einem der Ansprüche 2 bis 5 oder ein pharmazeutisch annehmbares Salz davon und gegebenenfalls weiterhin ein pharmazeutisches Trägermaterial umfaßt.

**Revendications**

1. Composés répondant à la formule développée I

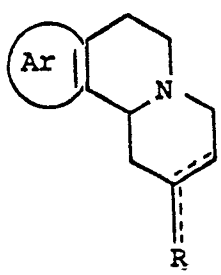

I

ou les sels des composés de formule I, où Ar représente un groupe aromatique ou hétéroaromatique choisi parmi

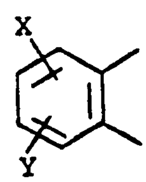

benzo-

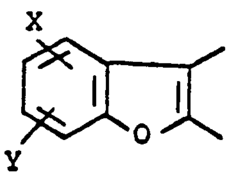

benzofuro-

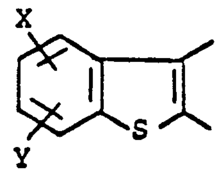

benzothieno-

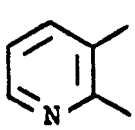

pyridino-

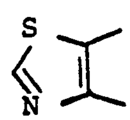

thiazolo-

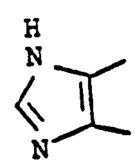

imidazo-

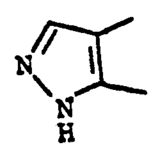

pyrazolo-

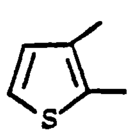

thieno-

et

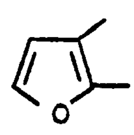

furo-

où

X et Y sont indépendamment:

1) hydrogène,
2) halogéno,
3) hydroxy,
4) Alcoxy en $C_{1-3}$, ou
5) alkyle en $C_{1-6}$;

R est:

1) $COOR^1$ où $R^1$ est un hydrogène ou un alkyle en $C_{1-5}$ à chaîne droite ou ramifée;

2) 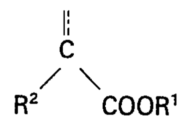

où

$R^1$ est comme défini en (1) et
$R^2$ est a) hydrogène,
b) alkyle en $C_{1-5}$, ou
c) allyle

3) 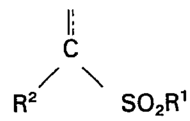

où

$R^1$ et $R^2$ sont comme défini en (2)

4) 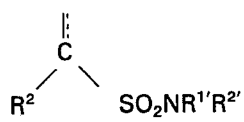

où

$R^{1'}$ et $R^{2'}$ ont la même signification que $R^1$ et $R^2$ en (2) ou
$R^{1'}$ et $R^{2'}$ forment ensemble, avec l'atome d'azote auquel ils sont fixés, un hétérocycle à 5 ou 6 chaînons qui est choisi dans le groupe comprenant pyrrolidino, morpholino, pipéridino et pipérazino;

5) 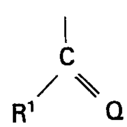

où

Q est un oxygène ou un groupe $=N—OR^1$ où $R^1$ a la même signification qu'en (1)

6) 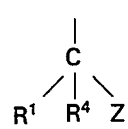

où

Z est a) $—OR^3$ ou $—SR^3$

où

$R^3$ est
i) H,
ii) alkyle en $C_{1-5}$ non substitué ou substitué par

1) —OH,
2) $—COOR^1$,
3) $—SO_2R^2$,
4) $—N(R^2)SO_2R^2$,

où

$R^1$ et $R^2$ sont comme défini en (2) ou
b) $—NR^2R^3$

où

$R^2$ est comme défini en (2) et
$R^3$ est comme défini en (a) ci-dessus
$R^4$ est a) hydrogène,
b) alkyle en $C_{1-5}$, ou
c) alkylidène en $C_{1-5}$ ou allyle et
$R^1$ est comme défini en (2) et les pointillés représentent des doubles liaisons éventuelles.

2. Composés selon la revendication 1, caractérisés en ce que, dans la formule I, Ar est un X,Y-benzo-, un X,Y-benzo[b]furo- ou un X,Y-benzo[b]thiéno- ou les sels desdits composés.

3. Composés selon la revendication 2, caractérisés en ce que Ar est un benzo[b]furo.

4. Composés selon l'une quelconque des revendications 1 à 3, caractérisés en ce que, dans la formule I, R est un des groupes suivants

$$-CH_2-CO_2R^1,\quad CH_3\overset{|}{C}HCO_2R^1,\quad R^1-\overset{|}{C}=NOR^1,\quad R^1(R^4)\overset{|}{C}-OH,$$

$$R^1-\overset{|}{C}H-O(CH_2)_2OH, \text{ ou } \quad R^1(R^4)\overset{|}{C}-O-(CH_2)_2OH \text{ où}$$

$R^1$ et $R^4$ sont indépendamment des atomes d'hydrogène ou des groupes alkyles ayant 1 à 5 atomes de carbone ou les sels desdits composés.

5. Composés selon la revendication 4, caractérisées en ce que, dans les groupes répondant aux formules indiquées, $R^1$ et $R^4$ sont un hydrogène ou un méthyle, de préférence un méthyle, ou les sels desdits composés.

6. Procédé pour la préparation des composés de formule Ia

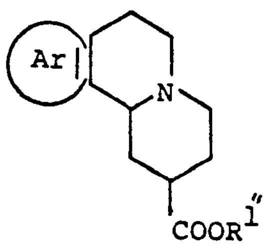

Ia

ou leurs sels où

Ar est comme défini dans la formule I de la revendication 1 et $R^{1''}$ est un alkyle en $C_{1-5}$ caractérisé en ce que l'on fait réagir un composé de formule II

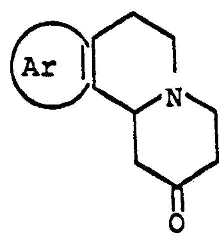

II

avec un 2-triméthylsilyl-1,3-dithianne de formule III

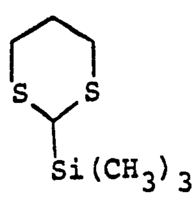

III

en présence d'un solvant de type éther et d'une base forte, pour obtenir un dérivé de 1,3-dithianne-2-ylidène de formule IV

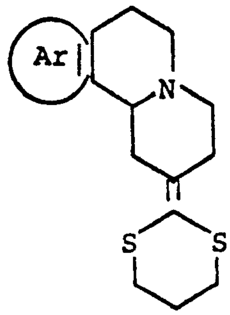

IV

que l'on fait réagir avec un alcanol en $C_{1-5}$ de formule $R^{1''}$—OH, lequel alcanol est saturé de chlorure d'hydrogène, pour obtenir les produits finals désirés de formule Ia, que l'on isole sous la forme libre ou sous forme de leurs sels.

7. Procédé pour la préparation des composés de formule I selon la revendication 1, caractérisé en ce que l'on prépare des composés de formule Ib

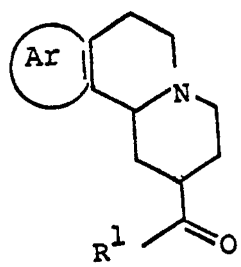

Ib

ou leurs sels où

Ar et $R^1$ ont la même signification que dans la revendication 1, caractérisé en ce que l'on fait réagir un composé de formule II

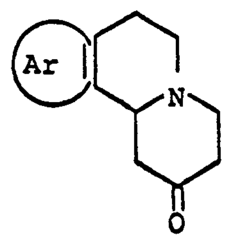

II

avec un composé phosphoré de formule

(alk $O)_2P=O$
|
CH
/ \
$R^1$ $SC_2H_5$

où

$R^1$ est un hydrogène ou un alkyle en $C_{1-5}$, ou on prépare les composés de formule Ib où $R^1$ est un hydrogène, par réaction de composé de formule II avec un composé phosphoré de formule

$$Ph_3P^+—CH_2—O—CH_3Cl^-$$

et où les produits intermédiaires produits par réaction avec lesdits composés phosphorés sont hydrolysés pour fournir les composés de formule Ib, et où l'hydrolyse est effectuée avec un acide minéral dilué lorsque le composé phosphoré utilisé est le composé triphénylphosphoro, et l'hydrolyse est effectuée avec du chlorure d'ammonium aqueux saturé sous réserve que le composé phosphoré soit un dialkylphosphonate de (1-éthylthio)alkyle et où les composés de formule Ib sont isolés sous la forme libre ou sous forme de leurs sels, et lesquels composés de formule Ib sont facultativement mis de plus à réagir avec un réactif de Grignard de formule $R^{4'}MgHal$ dans laquelle

$R^{4'}$ est un alkyle en $C_{1-5}$, un alkylidène en $C_{1-5}$ ou un allyle et

Hal est un halogène
pour fournir les composés de formule Ic

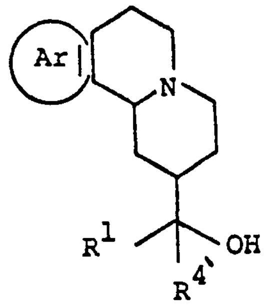

Ic

lesquels composés de formule Ic sont isolés sous la forme libre ou sous forme de leurs sels ou en ce que facultativement les composés céto de formule Ib sont de plus mis à réagir avec l'hydroxylamine ou une alcoxyamine de formule

$$H_2NOR^1$$

en présence d'une base organique, pour fournir les composés de formule Id

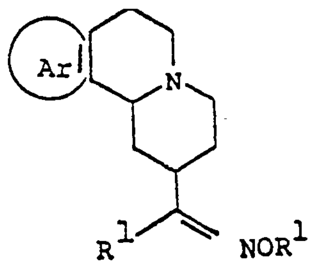

Id

lesquels composés de formule Id sont isolés sous la forme libre ou sous forme de leurs sels, ou en ce que, facultativement, les composés céto de formule Ib sont ensuite réduits, de préférence avec un hydrure métallique complexe, pour fournir les composés de formule Ie

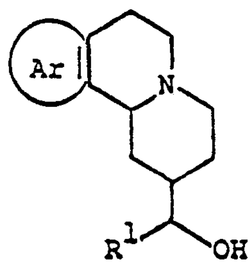

Ie

lesquels composés de formule Ie sont isolés sous la forme libre ou sous forme de leurs sels ou en ce que, facultativement, les composés céto de formule Ib sont de plus soumis à une amination par réduction avec une amine de formule

$$R^2—NH—R^3$$

dans laquelle $R^2$ est un hydrogène et $R^3$ est un alkyle en $C_{1-5}$ ou un hydroxyéthyle, laquelle amination par réduction est de préférence effectuée en présence d'un cyanoborohydrure et de méthanol contenant du chlorure d'hydrogène, et les composés amino correspondants sont isolés sous la forme libre ou sous forme de leurs sels.

8. Procédé pour la préparation des composés de formule If

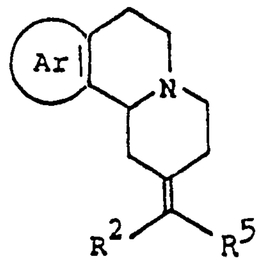

If

selon la revendication 1 ou de leurs sels
où
$R^5$ est $-COOR^1$,
$-SO_2R^1$ ou
$-SO_2NR^{1'}R^{2'}$
où
Ar, $R^1$, $R^2$, $R^{1'}$ et $R^{2'}$ ont la même signification que dans la revendication 1 caractérisé en ce que l'on fait réagir un composé de formule II

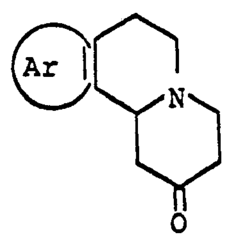

II

avec un réactif de Wittig, de préférence de formule

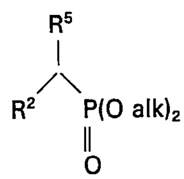

dans un solvant aprotique inerte en présence d'une base forte, pour fournir les composés de formule If que l'on isole sous forme des composés libres ou de leurs sels et où les composés de formule If sont facultativement de plus réduits, de préférence par hydrogénation en présence d'un catalyseur à métal noble, pour fournir les composés Ig

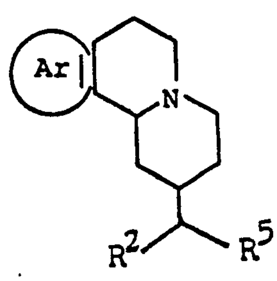

Ig

que l'on isole sous la forme libre ou sous forme de leurs sels.

9. Composition pharmaceutique pour le traitement de la dépression, de l'hypertension, de l'hypertension oculaire, du diabète, de l'obésité ou de l'agrégation plaquettaire ou pour modifier la motilité gastro-intestinale, caractérisée en ce qu'elle comprend, comme ingrédient pharmaceutiquement efficace, un composé de formule I selon la revendication 1 ou un sel pharmaceutiquement acceptable de celui-ci.

10. Composition pharmaceutique selon la revendication 9, caractérisée en ce qu'elle comprend, comme ingrédient actif, un composé selon l'une quelconque des revendications 2 à 5 ou un sel pharmaceutiquement acceptable de celui-ci et, facultativement, de plus un véhicule pharmaceutique.